# EUROPEAN PATENT SPECIFICATION

(11) **EP 1 879 611 B1**
(45) Date of publication and mention of the grant of the patent: **26.10.2011**
(21) Application number: 06754768.7
(22) Date of filing: 20.04.2006
(51) Int. Cl.: A61K 38/19, A61P 35/00, A61P 19/00

(54) **INSP163 POLYPEPTIDES AND POLYNUCLEOTIDES FOR THE TREATMENT OR PREVENTION OF LUNG CANCER**
INSP163-POLYPEPTIDE UND -POLYNUKLEOTIDE FÜR DIE BEHANDLUNG ODER VORBEUGUNG VON LUNGENKREBS
POLYPEPTIDES ET POLYNUCLEOTIDES INSP163 POUR LE TRAITEMENT OU LA PRÉVENTION DU CANCER DU POUMON

(30) Priority: 26.04.2005 EP 05103402; 17.05.2005 US 681731 P
(43) Date of publication of application: 23.01.2008
(73) Proprietor: Merck Serono SA, 1267 Coinsins, Vaud (CH)
(72) Inventor: POWER, Christine, F-01710 Thoiry (FR); YORKE-SMITH, Melanie, CH - Petit Lancy - Geneva (CH)
(86) International application number: PCT/EP2006/061710
(87) International publication number: WO 2006/114387

(56) References cited:
- WO-A-02/20569
- WO-A-03/031586
- WO-A-2005/042576
- KISHORE U ET AL: "CLQ: STRUCTURE, FUNCTION, AND RECEPTORS" IMMUNOPHARMACOLOGY, ELSEVIER SCIENCE PUBLISHERS BV, vol. 49, no. 1/2, August 2000 (2000-08), pages 159-170, XP001078864 ISSN: 0162-3109
- SHAPIRO LAWRENCE ET AL: "The crystal structure of a complement-1q family protein suggests an evolutionary link to tumor necrosis factor" CURRENT BIOLOGY, CURRENT SCIENCE,, GB, vol. 8, no. 6, 12 March 1998 (1998-03-12), pages 335-338, XP002148729 ISSN: 0960-9822

### SEQUENCE LISTING

<110> Applied Research systems ARS Holding N.V.
<120> NEW TREATMENT AND/OR PREVENTION OF CANCER AND/OR ARTHRITIS
<130> WO106
<150> European Patent Application No. 05103402.3
<151> 2005-04-26
<160> 50
<170> Seqwin99, version 1.02
<210> 1
   <211> 846
   <212> DNA
   <213> Homo sapiens
<400> 1
   gggggcgtcg gggcccggcg ggaggcacag aggacgcagc agcctggcca gcgcgcagat    60
   ccccccaacg ccaccgccag cgcgtcctcc cgcgaggggc tgcccgaggc ccccaagcca   120
   tcccaggcct caggacctga gttctccgac gcccacatga catggctgaa ctttgtccgg   180
   cggccggacg acggcgcctt aaggaagcgg tgcggaagca gggacaagaa gccgcgggat   240
   ctcttcggtc ccccaggacc tccaggtgca gaagtgaccg cggagactct gcttcacgag   300
   tttcaggagc tgctgaaaga ggccacggag cgccggttct cagggcttct ggacccgctg   360
   ctgccccagg gggcgggcct gcggctggtg ggcgaggcct ttcactgccg gctgcagggt   420
   ccccgccggg tggacaagcg gacgctggtg gagctgcatg gtttccaggc tcctgctgcc   480
   caaggtgcct tcctgcgagg ctccggtctg agcctggcct cgggtcggtt cacggccccc   540
   gtgtccggca tcttccagtt ctctgccagt ctgcacgtgg accacagtga gctgcagggc   600
   aaggcccggc tgcgggcccg ggacgtggtg tgtgttctca tctgtattga gtccctgtgc   660
   cagcgccaca cgtgcctgga ggccgtctca ggcctggaga gcaacagcag ggtcttcacg   720
   ctacaggtgc aggggctgct gcagctgcag gctggacagt acgcttctgt gtttgtggac   780
   aatggctccg gggccgtcct caccatccag gcgggctcca gcttctccgg gctgctcctg   840
   ggcacg                                                               846
<210> 2
   <211> 282
   <212> PRT
   <213> Homo sapiens
<400> 2
   Gly Gly Val Gly Ala Arg Arg Glu Ala Gln Arg Thr Gln Gln Pro Gly
   1               5                   10                  15
   Gln Arg Ala Asp Pro Pro Asn Ala Thr Ala Ser Ala Ser Ser Arg Glu
               20                  25                  30
   Gly Leu Pro Glu Ala Pro Lys Pro Ser Gln Ala Ser Gly Pro Glu Phe
           35                  40                  45
   Ser Asp Ala His Met Thr Trp Leu Asn Phe Val Arg Arg Pro Asp Asp
       50                  55                  60
   Gly Ala Leu Arg Lys Arg Cys Gly Ser Arg Asp Lys Lys Pro Arg Asp
   65                  70                  75                  80
   Leu Phe Gly Pro Pro Gly Pro Pro Gly Ala Glu Val Thr Ala Glu Thr
                   85                  90                  95
   Leu Leu His Glu Phe Gln Glu Leu Leu Lys Glu Ala Thr Glu Arg Arg
               100                 105                 110
   Phe Ser Gly Leu Leu Asp Pro Leu Leu Pro Gln Gly Ala Gly Leu Arg
           115                 120                 125
   Leu Val Gly Glu Ala Phe His Cys Arg Leu Gln Gly Pro Arg Arg Val
       130                 135                 140
   Asp Lys Arg Thr Leu Val Glu Leu His Gly Phe Gln Ala Pro Ala Ala
   145                 150                 155                 160
   Gln Gly Ala Phe Leu Arg Gly Ser Gly Leu Ser Leu Ala Ser Gly Arg
                   165                 170                 175
   Phe Thr Ala Pro Val Ser Gly Ile Phe Gln Phe Ser Ala Ser Leu His
               180                 185                 190
   Val Asp His Ser Glu Leu Gln Gly Lys Ala Arg Leu Arg Ala Arg Asp
           195                 200                 205
   Val Val Cys Val Leu Ile Cys Ile Glu Ser Leu Cys Gln Arg His Thr
       210                 215                 220
   Cys Leu Glu Ala Val Ser Gly Leu Glu Ser Asn Ser Arg Val Phe Thr
   225                 230                 235                 240
   Leu Gln Val Gln Gly Leu Leu Gln Leu Gln Ala Gly Gln Tyr Ala Ser
                   245                 250                 255
   Val Phe Val Asp Asn Gly Ser Gly Ala Val Leu Thr Ile Gln Ala Gly
               260                 265                 270
   Ser Ser Phe Ser Gly Leu Leu Leu Gly Thr
           275                 280
<210> 3
   <211> 663
   <212> DNA
   <213> Homo sapiens
<400> 3
   ccggacgacg gcgccttaag gaagcggtgc ggaagcaggg acaagaagcc gcgggatctc      60
   ttcggtcccc caggacctcc aggtgcagaa gtgaccgcgg agactctgct tcacgagttt     120
   caggagctgc tgaaagaggc cacggagcgc cggttctcag ggcttctgga cccgctgctg     180
   ccccaggggg cgggcctgcg gctggtgggc gaggcctttc actgccggct gcagggtccc     240
   cgccgggtgg acaagcggac gctggtggag ctgcatggtt tccaggctcc tgctgcccaa     300
   ggtgccttcc tgcgaggctc cggtctgagc ctggcctcgg gtcggttcac ggcccccgtg     360
   tccggcatct tccagttctc tgccagtctg cacgtggacc acagtgagct gcagggcaag     420
   gcccggctgc gggcccggga cgtggtgtgt gttctcatct gtattgagtc cctgtgccag     480
   cgccacacgt gcctggaggc cgtctcaggc ctggagagca acagcagggt cttcacgcta     540
   caggtgcagg ggctgctgca gctgcaggct ggacagtacg cttctgtgtt tgtggacaat     600
   ggctccgggg ccgtcctcac catccaggcg ggctccagct tctccgggct gctcctgggc     660
   acg                                                                   663
<210> 4
   <211> 221
   <212> PRT
   <213> Homo sapiens
<400> 4
   Pro Asp Asp Gly Ala Leu Arg Lys Arg Cys Gly Ser Arg Asp Lys Lys
   1               5                   10                  15
   Pro Arg Asp Leu Phe Gly Pro Pro Gly Pro Pro Gly Ala Glu Val Thr
               20                  25                  30
   Ala Glu Thr Leu Leu His Glu Phe Gln Glu Leu Leu Lys Glu Ala Thr
           35                  40                  45
   Glu Arg Arg Phe Ser Gly Leu Leu Asp Pro Leu Leu Pro Gln Gly Ala
       50                  55                  60
   Gly Leu Arg Leu Val Gly Glu Ala Phe His Cys Arg Leu Gln Gly Pro
   65                  70                  75                  80
   Arg Arg Val Asp Lys Arg Thr Leu Val Glu Leu His Gly Phe Gln Ala
                   85                  90                  95
   Pro Ala Ala Gln Gly Ala Phe Leu Arg Gly Ser Gly Leu Ser Leu Ala
               100                 105                 110
   Ser Gly Arg Phe Thr Ala Pro Val Ser Gly Ile Phe Gln Phe Ser Ala
           115                 120                 125
   Ser Leu His Val Asp His Ser Glu Leu Gln Gly Lys Ala Arg Leu Arg
       130                 135                 140
   Ala Arg Asp Val Val Cys Val Leu Ile Cys Ile Glu Ser Leu Cys Gln
   145                 150                 155                 160
   Arg His Thr Cys Leu Glu Ala Val Ser Gly Leu Glu Ser Asn Ser Arg
                   165                 170                 175
   Val Phe Thr Leu Gln Val Gln Gly Leu Leu Gln Leu Gln Ala Gly Gln
               180                 185                 190
   Tyr Ala Ser Val Phe Val Asp Asn Gly Ser Gly Ala Val Leu Thr Ile
           195                 200                 205
   Gln Ala Gly Ser Ser Phe Ser Gly Leu Leu Leu Gly Thr
       210                 215                 220
<210> 5
   <211> 642
   <212> DNA
   <213> Homo sapiens
<400> 5
   aagcggtgcg gaagcaggga caagaagccg cgggatctct tcggtccccc aggacctcca   60
   ggtgcagaag tgaccgcgga gactctgctt cacgagtttc aggagctgct gaaagaggcc  120
   acggagcgcc ggttctcagg gcttctggac ccgctgctgc cccagggggc gggcctgcgg  180
   ctggtgggcg aggcctttca ctgccggctg cagggtcccc gccgggtgga caagcggacg  240
   ctggtggagc tgcatggttt ccaggctcct gctgcccaag gtgccttcct gcgaggctcc  300
   ggtctgagcc tggcctcggg tcggttcacg gcccccgtgt ccggcatctt ccagttctct  360
   gccagtctgc acgtggacca cagtgagctg cagggcaagg cccggctgcg ggcccgggac  420
   gtggtgtgtg ttctcatctg tattgagtcc ctgtgccagc gccacacgtg cctggaggcc  480
   gtctcaggcc tggagagcaa cagcagggtc ttcacgctac aggtgcaggg gctgctgcag  540
   ctgcaggctg gacagtacgc ttctgtgttt gtggacaatg gctccggggc cgtcctcacc  600
   atccaggcgg gctccagctt ctccgggctg ctcctgggca cg                     642
<210> 6
   <211> 214
   <212> PRT
   <213> Homo sapiens
<400> 6
   Lys Arg Cys Gly Ser Arg Asp Lys Lys Pro Arg Asp Leu Phe Gly Pro
   1               5                   10                  15
   Pro Gly Pro Pro Gly Ala Glu Val Thr Ala Glu Thr Leu Leu His Glu
               20                  25                  30
   Phe Gln Glu Leu Leu Lys Glu Ala Thr Glu Arg Arg Phe Ser Gly Leu
           35                  40                  45
   Leu Asp Pro Leu Leu Pro Gln Gly Ala Gly Leu Arg Leu Val Gly Glu
       50                  55                  60
   Ala Phe His Cys Arg Leu Gln Gly Pro Arg Arg Val Asp Lys Arg Thr
   65                  70                  75                  80
   Leu Val Glu Leu His Gly Phe Gln Ala Pro Ala Ala Gln Gly Ala Phe
                   85                  90                  95
   Leu Arg Gly Ser Gly Leu Ser Leu Ala Ser Gly Arg Phe Thr Ala Pro
               100                 105                 110
   Val Ser Gly Ile Phe Gln Phe Ser Ala Ser Leu His Val Asp His Ser
           115                 120                 125
   Glu Leu Gln Gly Lys Ala Arg Leu Arg Ala Arg Asp Val Val Cys Val
       130                 135                 140
   Leu Ile Cys Ile Glu Ser Leu Cys Gln Arg His Thr Cys Leu Glu Ala
   145                 150                 155                 160
   Val Ser Gly Leu Glu Ser Asn Ser Arg Val Phe Thr Leu Gln Val Gln
                   165                 170                 175
   Gly Leu Leu Gln Leu Gln Ala Gly Gln Tyr Ala Ser Val Phe Val Asp
               180                 185                 190
   Asn Gly Ser Gly Ala Val Leu Thr Ile Gln Ala Gly Ser Ser Phe Ser
           195                 200                 205
   Gly Leu Leu Leu Gly Thr
       210
<210> 7
   <211> 636
   <212> DNA
   <213> Homo sapiens
<400> 7
   tgcggaagca gggacaagaa gccgcgggat ctcttcggtc ccccaggacc tccaggtgca    60
   gaagtgaccg cggagactct gcttcacgag tttcaggagc tgctgaaaga ggccacggag   120
   cgccggttct cagggcttct ggacccgctg ctgccccagg gggcgggcct gcggctggtg   180
   ggcgaggcct ttcactgccg gctgcagggt ccccgccggg tggacaagcg gacgctggtg   240
   gagctgcatg gtttccaggc tcctgctgcc caaggtgcct tcctgcgagg ctccggtctg   300
   agcctggcct cgggtcggtt cacggccccc gtgtccggca tcttccagtt ctctgccagt   360
   ctgcacgtgg accacagtga gctgcagggc aaggcccggc tgcgggcccg ggacgtggtg   420
   tgtgttctca tctgtattga gtccctgtgc cagcgccaca cgtgcctgga ggccgtctca   480
   ggcctggaga gcaacagcag ggtcttcacg ctacaggtgc aggggctgct gcagctgcag   540
   gctggacagt acgcttctgt gtttgtggac aatggctccg gggccgtcct caccatccag    600
   gcgggctcca gcttctccgg gctgctcctg ggcacg                              636
<210> 8
   <211> 212
   <212> PRT
   <213> Homo sapiens
<400> 8
   Cys Gly Ser Arg Asp Lys Lys Pro Arg Asp Leu Phe Gly Pro Pro Gly
   1               5                   10                  15
   Pro Pro Gly Ala Glu Val Thr Ala Glu Thr Leu Leu His Glu Phe Gln
               20                  25                  30
   Glu Leu Leu Lys Glu Ala Thr Glu Arg Arg Phe Ser Gly Leu Leu Asp
           35                  40                  45
   Pro Leu Leu Pro Gln Gly Ala Gly Leu Arg Leu Val Gly Glu Ala Phe
       50                  55                  60
   His Cys Arg Leu Gln Gly Pro Arg Arg Val Asp Lys Arg Thr Leu Val
   65                  70                  75                  80
   Glu Leu His Gly Phe Gln Ala Pro Ala Ala Gln Gly Ala Phe Leu Arg
                   85                  90                  95
   Gly Ser Gly Leu Ser Leu Ala Ser Gly Arg Phe Thr Ala Pro Val Ser
               100                 105                 110
   Gly Ile Phe Gln Phe Ser Ala Ser Leu His Val Asp His Ser Glu Leu
           115                 120                 125
   Gln Gly Lys Ala Arg Leu Arg Ala Arg Asp Val Val Cys Val Leu Ile
       130                 135                 140
   Cys Ile Glu Ser Leu Cys Gln Arg His Thr Cys Leu Glu Ala Val Ser
   145                 150                 155                 160
   Gly Leu Glu Ser Asn Ser Arg Val Phe Thr Leu Gln Val Gln Gly Leu
                   165                 170                 175
   Leu Gln Leu Gln Ala Gly Gln Tyr Ala Ser Val Phe Val Asp Asn Gly
               180                 185                 190
   Ser Gly Ala Val Leu Thr Ile Gln Ala Gly Ser Ser Phe Ser Gly Leu
           195                 200                 205
   Leu Leu Gly Thr
       210
<210> 9
   <211> 510
   <212> DNA
   <213> Homo sapiens
<400> 9
   ttctcagggc ttctggaccc gctgctgccc cagggggcgg gcctgcggct ggtgggcgag 60
   gcctttcact gccggctgca gggtccccgc cgggtggaca agcggacgct ggtggagctg 120
   catggtttcc aggctcctgc tgcccaaggt gccttcctgc gaggctccgg tctgagcctg 180
   gcctcgggtc ggttcacggc ccccgtgtcc ggcatcttcc agttctctgc cagtctgcac 240
   gtggaccaca gtgagctgca gggcaaggcc cggctgcggg cccgggacgt ggtgtgtgtt 300
   ctcatctgta ttgagtccct gtgccagcgc cacacgtgcc tggaggccgt ctcaggcctg 360
   gagagcaaca gcagggtctt cacgctacag gtgcaggggc tgctgcagct gcaggctgga 420
   cagtacgctt ctgtgtttgt ggacaatggc tccggggccg tcctcaccat ccaggcgggc 480
   tccagcttct ccgggctgct cctgggcacg 510
<210> 10
   <211> 170
   <212> PRT
   <213> Homo sapiens
<400> 10
   Phe Ser Gly Leu Leu Asp Pro Leu Leu Pro Gln Gly Ala Gly Leu Arg
   1               5                   10                  15
   Leu Val Gly Glu Ala Phe His Cys Arg Leu Gln Gly Pro Arg Arg Val
               20                  25                  30
   Asp Lys Arg Thr Leu Val Glu Leu His Gly Phe Gln Ala Pro Ala Ala
           35                  40                  45
   Gln Gly Ala Phe Leu Arg Gly Ser Gly Leu Ser Leu Ala Ser Gly Arg
       50                  55                  60
   Phe Thr Ala Pro Val Ser Gly Ile Phe Gln Phe Ser Ala Ser Leu His
   65                  70                  75                  80
   Val Asp His Ser Glu Leu Gln Gly Lys Ala Arg Leu Arg Ala Arg Asp
                   85                  90                  95
   Val Val Cys Val Leu Ile Cys Ile Glu Ser Leu Cys Gln Arg His Thr
               100                 105                 110
   Cys Leu Glu Ala Val Ser Gly Leu Glu Ser Asn Ser Arg Val Phe Thr
           115                 120                 125
   Leu Gln Val Gln Gly Leu Leu Gln Leu Gln Ala Gly Gln Tyr Ala Ser
       130                 135                 140
   Val Phe Val Asp Asn Gly Ser Gly Ala Val Leu Thr Ile Gln Ala Gly
   145                 150                 155                 160
   Ser Ser Phe Ser Gly Leu Leu Leu Gly Thr
                   165                 170
<210> 11
   <211> 417
   <212> DNA
   <213> Homo sapiens
<400> 11
   gtggacaagc ggacgctggt ggagctgcat ggtttccagg ctcctgctgc ccaaggtgcc     60
   ttcctgcgag gctccggtct gagcctggcc tcgggtcggt tcacggcccc cgtgtccggc    120
   atcttccagt tctctgccag tctgcacgtg gaccacagtg agctgcaggg caaggcccgg    180
   ctgcgggccc gggacgtggt gtgtgttctc atctgtattg agtccctgtg ccagcgccac    240
   acgtgcctgg aggccgtctc aggcctggag agcaacagca gggtcttcac gctacaggtg    300
   caggggctgc tgcagctgca ggctggacag tacgcttctg tgtttgtgga caatggctcc    360
   ggggccgtcc tcaccatcca ggcgggctcc agcttctccg ggctgctcct gggcacg       417
<210> 12
   <211> 139
   <212> PRT
   <213> Homo sapiens
<400> 12
   Val Asp Lys Arg Thr Leu Val Glu Leu His Gly Phe Gln Ala Pro Ala
   1               5                   10                  15
   Ala Gln Gly Ala Phe Leu Arg Gly Ser Gly Leu Ser Leu Ala Ser Gly
               20                  25                  30
   Arg Phe Thr Ala Pro Val Ser Gly Ile Phe Gln Phe Ser Ala Ser Leu
           35                  40                  45
   His Val Asp His Ser Glu Leu Gln Gly Lys Ala Arg Leu Arg Ala Arg
       50                  55                  60
   Asp Val Val Cys Val Leu Ile Cys Ile Glu Ser Leu Cys Gln Arg His
   65                  70                  75                  80
   Thr Cys Leu Glu Ala Val Ser Gly Leu Glu Ser Asn Ser Arg Val Phe
                   85                  90                  95
   Thr Leu Gln Val Gln Gly Leu Leu Gln Leu Gln Ala Gly Gln Tyr Ala
               100                 105                 110
   Ser Val Phe Val Asp Asn Gly Ser Gly Ala Val Leu Thr Ile Gln Ala
           115                 120                 125
   Gly Ser Ser Phe Ser Gly Leu Leu Leu Gly Thr
       130                 135
<210> 13
   <211> 405
   <212> DNA
   <213> Homo sapiens
<400> 13
   acgctggtgg agctgcatgg tttccaggct cctgctgccc aaggtgcctt cctgcgaggc 60
   tccggtctga gcctggcctc gggtcggttc acggcccccg tgtccggcat cttccagttc 120
   tctgccagtc tgcacgtgga ccacagtgag ctgcagggca aggcccggct gcgggcccgg 180
   gacgtggtgt gtgttctcat ctgtattgag tccctgtgcc agcgccacac gtgcctggag 240
   gccgtctcag gcctggagag caacagcagg gtcttcacgc tacaggtgca ggggctgctg 300
   cagctgcagg ctggacagta cgcttctgtg tttgtggaca atggctccgg ggccgtcctc 360
   accatccagg cgggctccag cttctccggg ctgctcctgg gcacg 405
<210> 14
<211> 135
   <212> PRT
   <213> Homo sapiens
<400> 14
   Thr Leu Val Glu Leu His Gly Phe Gln Ala Pro Ala Ala Gln Gly Ala
   1               5                   10                  15
   Phe Leu Arg Gly Ser Gly Leu Ser Leu Ala Ser Gly Arg Phe Thr Ala
               20                  25                  30
   Pro Val Ser Gly Ile Phe Gln Phe Ser Ala Ser Leu His Val Asp His
           35                  40                  45
   Ser Glu Leu Gln Gly Lys Ala Arg Leu Arg Ala Arg Asp Val Val Cys
       50                  55                  60
   Val Leu Ile Cys Ile Glu Ser Leu Cys Gln Arg His Thr Cys Leu Glu
   65                  70                  75                  80
   Ala Val Ser Gly Leu Glu Ser Asn Ser Arg Val Phe Thr Leu Gln Val
                   85                  90                  95
   Gln Gly Leu Leu Gln Leu Gln Ala Gly Gln Tyr Ala Ser Val Phe Val
               100                 105                 110
   Asp Asn Gly Ser Gly Ala Val Leu Thr Ile Gln Ala Gly Ser Ser Phe
           115                 120                 125
   Ser Gly Leu Leu Leu Gly Thr
       130                 135
<210> 15
   <211> 864
   <212> DNA
   <213> Homo sapiens
<400> 15
   gggggcgtcg gggcccggcg ggaggcacag aggacgcagc agcctggcca gcgcgcagat     60
   ccccccaacg ccaccgccag cgcgtcctcc cgcgaggggc tgcccgaggc ccccaagcca   120
   tcccaggcct caggacctga gttctccgac gcccacatga catggctgaa ctttgtccgg    180
   cggccggacg acggcgcctt aaggaagcgg tgcggaagca gggacaagaa gccgcgggat    240
   ctcttcggtc ccccaggacc tccaggtgca gaagtgaccg cggagactct gcttcacgag    300
   tttcaggagc tgctgaaaga ggccacggag cgccggttct cagggcttct ggacccgctg    360
   ctgccccagg gggcgggcct gcggctggtg ggcgaggcct ttcactgccg gctgcagggt    420
   ccccgccggg tggacaagcg gacgctggtg gagctgcatg gtttccaggc tcctgctgcc    480
   caaggtgcct tcctgcgagg ctccggtctg agcctggcct cgggtcggtt cacggccccc    540
   gtgtccggca tcttccagtt ctctgccagt ctgcacgtgg accacagtga gctgcagggc    600
   aaggcccggc tgcgggcccg ggacgtggtg tgtgttctca tctgtattga gtccctgtgc    660
   cagcgccaca cgtgcctgga ggccgtctca ggcctggaga gcaacagcag ggtcttcacg    720
   ctacaggtgc aggggctgct gcagctgcag gctggacagt acgcttctgt gtttgtggac    780
   aatggctccg gggccgtcct caccatccag gcgggctcca gcttctccgg gctgctcctg     840
   ggcacgcacc atcaccatca ccat                                            864
<210> 16
   <211> 288
   <212> PRT
   <213> Homo sapiens
<400> 16
   Gly Gly Val Gly Ala Arg Arg Glu Ala Gln Arg Thr Gln Gln Pro Gly
   1               5                   10                  15
   Gln Arg Ala Asp Pro Pro Asn Ala Thr Ala Ser Ala Ser Ser Arg Glu
               20                  25                  30
   Gly Leu Pro Glu Ala Pro Lys Pro Ser Gln Ala Ser Gly Pro Glu Phe
           35                  40                  45
   Ser Asp Ala His Met Thr Trp Leu Asn Phe Val Arg Arg Pro Asp Asp
       50                  55                  60
   Gly Ala Leu Arg Lys Arg Cys Gly Ser Arg Asp Lys Lys Pro Arg Asp
   65                  70                  75                  80
   Leu Phe Gly Pro Pro Gly Pro Pro Gly Ala Glu Val Thr Ala Glu Thr
                   85                  90                  95
   Leu Leu His Glu Phe Gln Glu Leu Leu Lys Glu Ala Thr Glu Arg Arg
               100                 105                 110
   Phe Ser Gly Leu Leu Asp Pro Leu Leu Pro Gln Gly Ala Gly Leu Arg
           115                 120                 125
   Leu Val Gly Glu Ala Phe His Cys Arg Leu Gln Gly Pro Arg Arg Val
       130                 135                 140
   Asp Lys Arg Thr Leu Val Glu Leu His Gly Phe Gln Ala Pro Ala Ala
   145                 150                 155                 160
   Gln Gly Ala Phe Leu Arg Gly Ser Gly Leu Ser Leu Ala Ser Gly Arg
                   165                 170                 175
   Phe Thr Ala Pro Val Ser Gly Ile Phe Gln Phe Ser Ala Ser Leu His
               180                 185                 190
   Val Asp His Ser Glu Leu Gln Gly Lys Ala Arg Leu Arg Ala Arg Asp
           195                 200                 205
   Val Val Cys Val Leu Ile Cys Ile Glu Ser Leu Cys Gln Arg His Thr
       210                 215                 220
   Cys Leu Glu Ala Val Ser Gly Leu Glu Ser Asn Ser Arg Val Phe Thr
   225                 230                 235                 240
   Leu Gln Val Gln Gly Leu Leu Gln Leu Gln Ala Gly Gln Tyr Ala Ser
                   245                 250                 255
   Val Phe Val Asp Asn Gly Ser Gly Ala Val Leu Thr Ile Gln Ala Gly
               260                 265                 270
   Ser Ser Phe Ser Gly Leu Leu Leu Gly Thr His His His His His His
           275                 280                 285
<210> 17
   <211> 681
   <212> DNA
   <213> Homo sapiens
<400> 17
   ccggacgacg gcgccttaag gaagcggtgc ggaagcaggg acaagaagcc gcgggatctc   60
   ttcggtcccc caggacctcc aggtgcagaa gtgaccgcgg agactctgct tcacgagttt  120
   caggagctgc tgaaagaggc cacggagcgc cggttctcag ggcttctgga cccgctgctg  180
   ccccaggggg cgggcctgcg gctggtgggc gaggcctttc actgccggct gcagggtccc  240
   cgccgggtgg acaagcggac gctggtggag ctgcatggtt tccaggctcc tgctgcccaa  300
   ggtgccttcc tgcgaggctc cggtctgagc ctggcctcgg gtcggttcac ggcccccgtg  360
   tccggcatct tccagttctc tgccagtctg cacgtggacc acagtgagct gcagggcaag  420
   gcccggctgc gggcccggga cgtggtgtgt gttctcatct gtattgagtc cctgtgccag  480
   cgccacacgt gcctggaggc cgtctcaggc ctggagagca acagcagggt cttcacgcta  540
   caggtgcagg ggctgctgca gctgcaggct ggacagtacg cttctgtgtt tgtggacaat  600
   ggctccgggg ccgtcctcac catccaggcg ggctccagct tctccgggct gctcctgggc  660
   acgcaccatc accatcacca t                                            681
<210> 18
   <211> 227
   <212> PRT
   <213> Homo sapiens
<400> 18
   Pro Asp Asp Gly Ala Leu Arg Lys Arg Cys Gly Ser Arg Asp Lys Lys
   1               5                   10                  15
   Pro Arg Asp Leu Phe Gly Pro Pro Gly Pro Pro Gly Ala Glu Val Thr
               20                  25                  30
   Ala Glu Thr Leu Leu His Glu Phe Gln Glu Leu Leu Lys Glu Ala Thr
           35                  40                  45
   Glu Arg Arg Phe Ser Gly Leu Leu Asp Pro Leu Leu Pro Gln Gly Ala
       50                  55                  60
   Gly Leu Arg Leu Val Gly Glu Ala Phe His Cys Arg Leu Gln Gly Pro
   65                  70                  75                  80
   Arg Arg Val Asp Lys Arg Thr Leu Val Glu Leu His Gly Phe Gln Ala
                   85                  90                  95
   Pro Ala Ala Gln Gly Ala Phe Leu Arg Gly Ser Gly Leu Ser Leu Ala
               100                 105                 110
   Ser Gly Arg Phe Thr Ala Pro Val Ser Gly Ile Phe Gln Phe Ser Ala
           115                 120                 125
   Ser Leu His Val Asp His Ser Glu Leu Gln Gly Lys Ala Arg Leu Arg
       130                 135                 140
   Ala Arg Asp Val Val Cys Val Leu Ile Cys Ile Glu Ser Leu Cys Gln
   145                 150                 155                 160
   Arg His Thr Cys Leu Glu Ala Val Ser Gly Leu Glu Ser Asn Ser Arg
                   165                 170                 175
   Val Phe Thr Leu Gln Val Gln Gly Leu Leu Gln Leu Gln Ala Gly Gln
               180                 185                 190
   Tyr Ala Ser Val Phe Val Asp Asn Gly Ser Gly Ala Val Leu Thr Ile
           195                 200                 205
   Gln Ala Gly Ser Ser Phe Ser Gly Leu Leu Leu Gly Thr His His His
       210                 215                 220
   His His His
   225
<210> 19
   <211> 660
   <212> DNA
   <213> Homo sapiens
<400> 19
   aagcggtgcg gaagcaggga caagaagccg cgggatctct tcggtccccc aggacctcca    60
   ggtgcagaag tgaccgcgga gactctgctt cacgagtttc aggagctgct gaaagaggcc   120
   acggagcgcc ggttctcagg gcttctggac ccgctgctgc cccagggggc gggcctgcgg   180
   ctggtgggcg aggcctttca ctgccggctg cagggtcccc gccgggtgga caagcggacg   240
   ctggtggagc tgcatggttt ccaggctcct gctgcccaag gtgccttcct gcgaggctcc   300
   ggtctgagcc tggcctcggg tcggttcacg gcccccgtgt ccggcatctt ccagttctct   360
   gccagtctgc acgtggacca cagtgagctg cagggcaagg cccggctgcg ggcccgggac   420
   gtggtgtgtg ttctcatctg tattgagtcc ctgtgccagc gccacacgtg cctggaggcc   480
   gtctcaggcc tggagagcaa cagcagggtc ttcacgctac aggtgcaggg gctgctgcag   540
   ctgcaggctg gacagtacgc ttctgtgttt gtggacaatg gctccggggc cgtcctcacc   600
   atccaggcgg gctccagctt ctccgggctg ctcctgggca cgcaccatca ccatcaccat   660
<210> 20
   <211> 220
   <212> PRT
   <213> Homo sapiens
<400> 20
   Lys Arg Cys Gly Ser Arg Asp Lys Lys Pro Arg Asp Leu Phe Gly Pro
   1               5                   10                  15
   Pro Gly Pro Pro Gly Ala Glu Val Thr Ala Glu Thr Leu Leu His Glu
               20                  25                  30
   Phe Gln Glu Leu Leu Lys Glu Ala Thr Glu Arg Arg Phe Ser Gly Leu
           35                  40                  45
   Leu Asp Pro Leu Leu Pro Gln Gly Ala Gly Leu Arg Leu Val Gly Glu
       50                  55                  60
   Ala Phe His Cys Arg Leu Gln Gly Pro Arg Arg Val Asp Lys Arg Thr
   65                  70                  75                  80
   Leu Val Glu Leu His Gly Phe Gln Ala Pro Ala Ala Gln Gly Ala Phe
                   85                  90                  95
   Leu Arg Gly Ser Gly Leu Ser Leu Ala Ser Gly Arg Phe Thr Ala Pro
               100                 105                 110
   Val Ser Gly Ile Phe Gln Phe Ser Ala Ser Leu His Val Asp His Ser
           115                 120                 125
   Glu Leu Gln Gly Lys Ala Arg Leu Arg Ala Arg Asp Val Val Cys Val
       130                 135                 140
   Leu Ile Cys Ile Glu Ser Leu Cys Gln Arg His Thr Cys Leu Glu Ala
   145                 150                 155                 160
   Val Ser Gly Leu Glu Ser Asn Ser Arg Val Phe Thr Leu Gln Val Gln
                   165                 170                 175
   Gly Leu Leu Gln Leu Gln Ala Gly Gln Tyr Ala Ser Val Phe Val Asp
               180                 185                 190
   Asn Gly Ser Gly Ala Val Leu Thr Ile Gln Ala Gly Ser Ser Phe Ser
           195                 200                 205
   Gly Leu Leu Leu Gly Thr His His His His His His
       210                 215                 220
<210> 21
   <211> 654
   <212> DNA
   <213> Homo sapiens
<400> 21
   tgcggaagca gggacaagaa gccgcgggat ctcttcggtc ccccaggacc tccaggtgca    60
   gaagtgaccg cggagactct gcttcacgag tttcaggagc tgctgaaaga ggccacggag   120
   cgccggttct cagggcttct ggacccgctg ctgccccagg gggcgggcct gcggctggtg   180
   ggcgaggcct ttcactgccg gctgcagggt ccccgccggg tggacaagcg gacgctggtg   240
   gagctgcatg gtttccaggc tcctgctgcc caaggtgcct tcctgcgagg ctccggtctg   300
   agcctggcct cgggtcggtt cacggccccc gtgtccggca tcttccagtt ctctgccagt   360
   ctgcacgtgg accacagtga gctgcagggc aaggcccggc tgcgggcccg ggacgtggtg   420
   tgtgttctca tctgtattga gtccctgtgc cagcgccaca cgtgcctgga ggccgtctca   480
   ggcctggaga gcaacagcag ggtcttcacg ctacaggtgc aggggctgct gcagctgcag   540
   gctggacagt acgcttctgt gtttgtggac aatggctccg gggccgtcct caccatccag    600
   gcgggctcca gcttctccgg gctgctcctg ggcacgcacc atcaccatca ccat         654
<210> 22
   <211> 218
   <212> PRT
   <213> Homo sapiens
<400> 22
   Cys Gly Ser Arg Asp Lys Lys Pro Arg Asp Leu Phe Gly Pro Pro Gly
   1               5                   10                  15
   Pro Pro Gly Ala Glu Val Thr Ala Glu Thr Leu Leu His Glu Phe Gln
               20                  25                  30
   Glu Leu Leu Lys Glu Ala Thr Glu Arg Arg Phe Ser Gly Leu Leu Asp
           35                  40                  45
   Pro Leu Leu Pro Gln Gly Ala Gly Leu Arg Leu Val Gly Glu Ala Phe
       50                  55                  60
   His Cys Arg Leu Gln Gly Pro Arg Arg Val Asp Lys Arg Thr Leu Val
   65                  70                  75                  80
   Glu Leu His Gly Phe Gln Ala Pro Ala Ala Gln Gly Ala Phe Leu Arg
                   85                  90                  95
   Gly Ser Gly Leu Ser Leu Ala Ser Gly Arg Phe Thr Ala Pro Val Ser
               100                 105                 110
   Gly Ile Phe Gln Phe Ser Ala Ser Leu His Val Asp His Ser Glu Leu
           115                 120                 125
   Gln Gly Lys Ala Arg Leu Arg Ala Arg Asp Val Val Cys Val Leu Ile
       130                 135                 140
   Cys Ile Glu Ser Leu Cys Gln Arg His Thr Cys Leu Glu Ala Val Ser
   145                 150                 155                 160
   Gly Leu Glu Ser Asn Ser Arg Val Phe Thr Leu Gln Val Gln Gly Leu
                   165                 170                 175
   Leu Gln Leu Gln Ala Gly Gln Tyr Ala Ser Val Phe Val Asp Asn Gly
               180                 185                 190
   Ser Gly Ala Val Leu Thr Ile Gln Ala Gly Ser Ser Phe Ser Gly Leu
           195                 200                 205
   Leu Leu Gly Thr His His His His His His
       210                 215
<210> 23
   <211> 528
   <212> DNA
   <213> Homo sapiens
<400> 23
   ttctcagggc ttctggaccc gctgctgccc cagggggcgg gcctgcggct ggtgggcgag    60
   gcctttcact gccggctgca gggtccccgc cgggtggaca agcggacgct ggtggagctg   120
   catggtttcc aggctcctgc tgcccaaggt gccttcctgc gaggctccgg tctgagcctg   180
   gcctcgggtc ggttcacggc ccccgtgtcc ggcatcttcc agttctctgc cagtctgcac    240
   gtggaccaca gtgagctgca gggcaaggcc cggctgcggg cccgggacgt ggtgtgtgtt   300
   ctcatctgta ttgagtccct gtgccagcgc cacacgtgcc tggaggccgt ctcaggcctg   360
   gagagcaaca gcagggtctt cacgctacag gtgcaggggc tgctgcagct gcaggctgga   420
   cagtacgctt ctgtgtttgt ggacaatggc tccggggccg tcctcaccat ccaggcgggc   480
   tccagcttct ccgggctgct cctgggcacg caccatcacc atcaccat                528
<210> 24
   <211> 176
   <212> PRT
   <213> Homo sapiens
<400> 24
   Phe Ser Gly Leu Leu Asp Pro Leu Leu Pro Gln Gly Ala Gly Leu Arg
   1               5                   10                  15
   Leu Val Gly Glu Ala Phe His Cys Arg Leu Gln Gly Pro Arg Arg Val
               20                  25                  30
   Asp Lys Arg Thr Leu Val Glu Leu His Gly Phe Gln Ala Pro Ala Ala
           35                  40                  45
   Gln Gly Ala Phe Leu Arg Gly Ser Gly Leu Ser Leu Ala Ser Gly Arg
       50                  55                  60
   Phe Thr Ala Pro Val Ser Gly Ile Phe Gln Phe Ser Ala Ser Leu His
   65                  70                  75                  80
   Val Asp His Ser Glu Leu Gln Gly Lys Ala Arg Leu Arg Ala Arg Asp
                   85                  90                  95
   Val Val Cys Val Leu Ile Cys Ile Glu Ser Leu Cys Gln Arg His Thr
               100                 105                 110
   Cys Leu Glu Ala Val Ser Gly Leu Glu Ser Asn Ser Arg Val Phe Thr
           115                 120                 125
   Leu Gln Val Gln Gly Leu Leu Gln Leu Gln Ala Gly Gln Tyr Ala Ser
       130                 135                 140
   Val Phe Val Asp Asn Gly Ser Gly Ala Val Leu Thr Ile Gln Ala Gly
   145                 150                 155                 160
   Ser Ser Phe Ser Gly Leu Leu Leu Gly Thr His His His His His His
                   165                 170                 175
<210> 25
   <211> 435
   <212> DNA
   <213> Homo sapiens
<400> 25
   gtggacaagc ggacgctggt ggagctgcat ggtttccagg ctcctgctgc ccaaggtgcc 60
   ttcctgcgag gctccggtct gagcctggcc tcgggtcggt tcacggcccc cgtgtccggc 120
   atcttccagt tctctgccag tctgcacgtg gaccacagtg agctgcaggg caaggcccgg 180
   ctgcgggccc gggacgtggt gtgtgttctc atctgtattg agtccctgtg ccagcgccac 240
   acgtgcctgg aggccgtctc aggcctggag agcaacagca gggtcttcac gctacaggtg 300
   caggggctgc tgcagctgca ggctggacag tacgcttctg tgtttgtgga caatggctcc 360
   ggggccgtcc tcaccatcca ggcgggctcc agcttctccg ggctgctcct gggcacgcac 420
   catcaccatc accat 435
<210> 26
   <211> 145
   <212> PRT
   <213> Homo sapiens
<400> 26
   Val Asp Lys Arg Thr Leu Val Glu Leu His Gly Phe Gln Ala Pro Ala
   1               5                   10                  15
   Ala Gln Gly Ala Phe Leu Arg Gly Ser Gly Leu Ser Leu Ala Ser Gly
               20                  25                  30
   Arg Phe Thr Ala Pro Val Ser Gly Ile Phe Gln Phe Ser Ala Ser Leu
           35                  40                  45
   His Val Asp His Ser Glu Leu Gln Gly Lys Ala Arg Leu Arg Ala Arg
       50                  55                  60
   Asp Val Val Cys Val Leu Ile Cys Ile Glu Ser Leu Cys Gln Arg His
   65                  70                  75                  80
   Thr Cys Leu Glu Ala Val Ser Gly Leu Glu Ser Asn Ser Arg Val Phe
                   85                  90                  95
   Thr Leu Gln Val Gln Gly Leu Leu Gln Leu Gln Ala Gly Gln Tyr Ala
               100                 105                 110
   Ser Val Phe Val Asp Asn Gly Ser Gly Ala Val Leu Thr Ile Gln Ala
           115                 120                 125
   Gly Ser Ser Phe Ser Gly Leu Leu Leu Gly Thr His His His His His
       130                 135                 140
   His
   145
<210> 27
   <211> 423
   <212> DNA
   <213> Homo sapiens
<400> 27
   acgctggtgg agctgcatgg tttccaggct cctgctgccc aaggtgcctt cctgcgaggc    60
   tccggtctga gcctggcctc gggtcggttc acggcccccg tgtccggcat cttccagttc   120
   tctgccagtc tgcacgtgga ccacagtgag ctgcagggca aggcccggct gcgggcccgg   180
   gacgtggtgt gtgttctcat ctgtattgag tccctgtgcc agcgccacac gtgcctggag   240
   gccgtctcag gcctggagag caacagcagg gtcttcacgc tacaggtgca ggggctgctg   300
   cagctgcagg ctggacagta cgcttctgtg tttgtggaca atggctccgg ggccgtcctc   360
   accatccagg cgggctccag cttctccggg ctgctcctgg gcacgcacca tcaccatcac   420
   cat                                                                 423
<210> 28
   <211> 141
   <212> PRT
   <213> Homo sapiens
<400> 28
   Thr Leu Val Glu Leu His Gly Phe Gln Ala Pro Ala Ala Gln Gly Ala
   1               5                   10                  15
   Phe Leu Arg Gly Ser Gly Leu Ser Leu Ala Ser Gly Arg Phe Thr Ala
               20                  25                  30
   Pro Val Ser Gly Ile Phe Gln Phe Ser Ala Ser Leu His Val Asp His
           35                  40                  45
   Ser Glu Leu Gln Gly Lys Ala Arg Leu Arg Ala Arg Asp Val Val Cys
       50                  55                  60
   Val Leu Ile Cys Ile Glu Ser Leu Cys Gln Arg His Thr Cys Leu Glu
   65                  70                  75                  80
   Ala Val Ser Gly Leu Glu Ser Asn Ser Arg Val Phe Thr Leu Gln Val
                   85                  90                  95
   Gln Gly Leu Leu Gln Leu Gln Ala Gly Gln Tyr Ala Ser Val Phe Val
               100                 105                 110
   Asp Asn Gly Ser Gly Ala Val Leu Thr Ile Gln Ala Gly Ser Ser Phe
           115                 120                 125
   Ser Gly Leu Leu Leu Gly Thr His His His His His His
       130                 135                 140
<210> 29
   <211> 906
   <212> DNA
   <213> Homo sapiens
<400> 29
   atgcggcgct gggcctgggc cgcggtcgtg gtcctcctcg ggccgcagct cgtgctcctc      60
   gggggcgtcg gggcccggcg ggaggcacag aggacgcagc agcctggcca gcgcgcagat     120
   ccccccaacg ccaccgccag cgcgtcctcc cgcgaggggc tgcccgaggc ccccaagcca    180
   tcccaggcct caggacctga gttctccgac gcccacatga catggctgaa ctttgtccgg     240
   cggccggacg acggcgcctt aaggaagcgg tgcggaagca gggacaagaa gccgcgggat     300
   ctcttcggtc ccccaggacc tccaggtgca gaagtgaccg cggagactct gcttcacgag     360
   tttcaggagc tgctgaaaga ggccacggag cgccggttct cagggcttct ggacccgctg     420
   ctgccccagg gggcgggcct gcggctggtg ggcgaggcct ttcactgccg gctgcagggt     480
   ccccgccggg tggacaagcg gacgctggtg gagctgcatg gtttccaggc tcctgctgcc     540
   caaggtgcct tcctgcgagg ctccggtctg agcctggcct cgggtcggtt cacggccccc     600
   gtgtccggca tcttccagtt ctctgccagt ctgcacgtgg accacagtga gctgcagggc     660
   aaggcccggc tgcgggcccg ggacgtggtg tgtgttctca tctgtattga gtccctgtgc     720
   cagcgccaca cgtgcctgga ggccgtctca ggcctggaga gcaacagcag ggtcttcacg     780
   ctacaggtgc aggggctgct gcagctgcag gctggacagt acgcttctgt gtttgtggac     840
   aatggctccg ggcccgtcct caccatccag gcgggctcca gcttctccgg gctgctcctg      900
   ggcacg                                                                906
<210> 30
   <211> 302
   <212> PRT
   <213> Homo sapiens
<400> 30
   Met Arg Arg Trp Ala Trp Ala Ala Val Val Val Leu Leu Gly Pro Gln
   1               5                   10                  15
   Leu Val Leu Leu Gly Gly Val Gly Ala Arg Arg Glu Ala Gln Arg Thr
               20                  25                  30
   Gln Gln Pro Gly Gln Arg Ala Asp Pro Pro Asn Ala Thr Ala Ser Ala
           35                  40                  45
   Ser Ser Arg Glu Gly Leu Pro Glu Ala Pro Lys Pro Ser Gln Ala Ser
       50                  55                  60
   Gly Pro Glu Phe Ser Asp Ala His Met Thr Trp Leu Asn Phe Val Arg
   65                  70                  75                  80
   Arg Pro Asp Asp Gly Ala Leu Arg Lys Arg Cys Gly Ser Arg Asp Lys
                   85                  90                  95
   Lys Pro Arg Asp Leu Phe Gly Pro Pro Gly Pro Pro Gly Ala Glu Val
               100                 105                 110
   Thr Ala Glu Thr Leu Leu His Glu Phe Gln Glu Leu Leu Lys Glu Ala
           115                 120                 125
   Thr Glu Arg Arg Phe Ser Gly Leu Leu Asp Pro Leu Leu Pro Gln Gly
       130                 135                 140
   Ala Gly Leu Arg Leu Val Gly Glu Ala Phe His Cys Arg Leu Gln Gly
   145                 150                 155                 160
   Pro Arg Arg Val Asp Lys Arg Thr Leu Val Glu Leu His Gly Phe Gln
                   165                 170                 175
   Ala Pro Ala Ala Gln Gly Ala Phe Leu Arg Gly Ser Gly Leu Ser Leu
               180                 185                 190
   Ala Ser Gly Arg Phe Thr Ala Pro Val Ser Gly Ile Phe Gln Phe Ser
           195                 200                 205
   Ala Ser Leu His Val Asp His Ser Glu Leu Gln Gly Lys Ala Arg Leu
       210                 215                 220
   Arg Ala Arg Asp Val Val Cys Val Leu Ile Cys Ile Glu Ser Leu Cys
   225                 230                 235                 240
   Gln Arg His Thr Cys Leu Glu Ala Val Ser Gly Leu Glu Ser Asn Ser
                   245                 250                 255
   Arg Val Phe Thr Leu Gln Val Gln Gly Leu Leu Gln Leu Gln Ala Gly
               260                 265                 270
   Gln Tyr Ala Ser Val Phe Val Asp Asn Gly Ser Gly Ala Val Leu Thr
           275                 280                 285
   Ile Gln Ala Gly Ser Ser Phe Ser Gly Leu Leu Leu Gly Thr
       290                 295                 300
<210> 31
   <211> 924
   <212> DNA
   <213> Homo sapiens
<400> 31
   atgcggcgct gggcctgggc cgcggtcgtg gtcctcctcg ggccgcagct cgtgctcctc    60
   gggggcgtcg gggcccggcg ggaggcacag aggacgcagc agcctggcca gcgcgcagat   120
   ccccccaacg ccaccgccag cgcgtcctcc cgcgaggggc tgcccgaggc ccccaagcca   180
   tcccaggcct caggacctga gttctccgac gcccacatga catggctgaa ctttgtccgg   240
   cggccggacg acggcgcctt aaggaagcgg tgcggaagca gggacaagaa gccgcgggat   300
   ctcttcggtc ccccaggacc tccaggtgca gaagtgaccg cggagactct gcttcacgag   360
   tttcaggagc tgctgaaaga ggccacggag cgccggttct cagggcttct ggacccgctg   420
   ctgccccagg gggcgggcct gcggctggtg ggcgaggcct ttcactgccg gctgcagggt   480
   ccccgccggg tggacaagcg gacgctggtg gagctgcatg gtttccaggc tcctgctgcc   540
   caaggtgcct tcctgcgagg ctccggtctg agcctggcct cgggtcggtt cacggccccc   600
   gtgtccggca tcttccagtt ctctgccagt ctgcacgtgg accacagtga gctgcagggc   660
   aaggcccggc tgcgggcccg ggacgtggtg tgtgttctca tctgtattga gtccctgtgc   720
   cagcgccaca cgtgcctgga ggccgtctca ggcctggaga gcaacagcag ggtcttcacg   780
   ctacaggtgc aggggctgct gcagctgcag gctggacagt acgcttctgt gtttgtggac   840
   aatggctccg ggccgtcctt caccatccag gcgggctcca gcttctccgg gctgctcctg    900
   ggcacgcacc atcaccatca ccat                                          924
<210> 32
   <211> 308
   <212> PRT
   <213> Homo sapiens
<400> 32
   Met Arg Arg Trp Ala Trp Ala Ala Val Val Val Leu Leu Gly Pro Gln
   1               5                   10                  15
   Leu Val Leu Leu Gly Gly Val Gly Ala Arg Arg Glu Ala Gln Arg Thr
               20                  25                  30
   Gln Gln Pro Gly Gln Arg Ala Asp Pro Pro Asn Ala Thr Ala Ser Ala
           35                  40                  45
   Ser Ser Arg Glu Gly Leu Pro Glu Ala Pro Lys Pro Ser Gln Ala Ser
       50                  55                  60
   Gly Pro Glu Phe Ser Asp Ala His Met Thr Trp Leu Asn Phe Val Arg
   65                  70                  75                  80
   Arg Pro Asp Asp Gly Ala Leu Arg Lys Arg Cys Gly Ser Arg Asp Lys
                   85                  90                  95
   Lys Pro Arg Asp Leu Phe Gly Pro Pro Gly Pro Pro Gly Ala Glu Val
               100                 105                 110
   Thr Ala Glu Thr Leu Leu His Glu Phe Gln Glu Leu Leu Lys Glu Ala
           115                 120                 125
   Thr Glu Arg Arg Phe Ser Gly Leu Leu Asp Pro Leu Leu Pro Gln Gly
       130                 135                 140
   Ala Gly Leu Arg Leu Val Gly Glu Ala Phe His Cys Arg Leu Gln Gly
   145                 150                 155                 160
   Pro Arg Arg Val Asp Lys Arg Thr Leu Val Glu Leu His Gly Phe Gln
                   165                 170                 175
   Ala Pro Ala Ala Gln Gly Ala Phe Leu Arg Gly Ser Gly Leu Ser Leu
   180 185 190
   Ala Ser Gly Arg Phe Thr Ala Pro Val Ser Gly Ile Phe Gln Phe Ser
   195 200 205
   Ala Ser Leu His Val Asp His Ser Glu Leu Gln Gly Lys Ala Arg Leu
   210 215 220
   Arg Ala Arg Asp Val Val Cys Val Leu Ile Cys Ile Glu Ser Leu Cys
   225 230 235 240
   Gln Arg His Thr Cys Leu Glu Ala Val Ser Gly Leu Glu Ser Asn Ser
   245 250 255
   Arg Val Phe Thr Leu Gln Val Gln Gly Leu Leu Gln Leu Gln Ala Gly
   260 265 270
   Gln Tyr Ala Ser Val Phe Val Asp Asn Gly Ser Gly Ala Val Leu Thr
   275 280 285
   Ile Gln Ala Gly Ser Ser Phe Ser Gly Leu Leu Leu Gly Thr His His
   290 295 300
   His His His His
   305
<210> 33
   <211> 831
   <212> DNA
   <213> Homo sapiens
<400> 33
   cggcgggagg cacagaggac gcagcagcct ggccagcgcg cagatccccc caacgccacc   60
   gccagcgcgt cctcccgcga ggggctgccc gaggccccca agccatccca ggcctcagga  120
   cctgagttct ccgacgccca catgacatgg ctgaactttg tccggcggcc ggacgacggc  180
   gccttaagga agcggtgcgg aagcagggac aagaagccgc gggatctctt cggtccccca  240
   ggacctccag gtgcagaagt gaccgcggag actctgcttc acgagtttca ggagctgctg  300
   aaagaggcca cggagcgccg gttctcaggg cttctggacc cgctgctgcc ccagggggcg 360
   ggcctgcggc tggtgggcga ggcctttcac tgccggctgc agggtccccg ccgggtggac  420
   aagcggacgc tggtggagct gcatggtttc caggctcctg ctgcccaagg tgccttcctg  480
   cgaggctccg gtctgagcct ggcctcgggt cggttcacgg cccccgtgtc cggcatcttc  540
   cagttctctg ccagtctgca cgtggaccac agtgagctgc agggcaaggc ccggctgcgg  600
   gcccgggacg tggtgtgtgt tctcatctgt attgagtccc tgtgccagcg ccacacgtgc  660
   ctggaggccg tctcaggcct ggagagcaac agcagggtct tcacgctaca ggtgcagggg  720
   ctgctgcagc tgcaggctgg acagtacgct tctgtgtttg tggacaatgg ctccggggcc  780
   gtcctcacca tccaggcggg ctccagcttc tccgggctgc tcctgggcac g            831
<210> 34
   <211> 277
   <212> PRT
   <213> Homo sapiens
<400> 34
   Arg Arg Glu Ala Gln Arg Thr Gln Gln Pro Gly Gln Arg Ala Asp Pro
   1               5                   10                  15
   Pro Asn Ala Thr Ala Ser Ala Ser Ser Arg Glu Gly Leu Pro Glu Ala
               20                  25                  30
   Pro Lys Pro Ser Gln Ala Ser Gly Pro Glu Phe Ser Asp Ala His Met
           35                  40                  45
   Thr Trp Leu Asn Phe Val Arg Arg Pro Asp Asp Gly Ala Leu Arg Lys
       50                  55                  60
   Arg Cys Gly Ser Arg Asp Lys Lys Pro Arg Asp Leu Phe Gly Pro Pro
   65                  70                  75                  80
   Gly Pro Pro Gly Ala Glu Val Thr Ala Glu Thr Leu Leu His Glu Phe
                   85                  90                  95
   Gln Glu Leu Leu Lys Glu Ala Thr Glu Arg Arg Phe Ser Gly Leu Leu
               100                 105                 110
   Asp Pro Leu Leu Pro Gln Gly Ala Gly Leu Arg Leu Val Gly Glu Ala
           115                 120                 125
   Phe His Cys Arg Leu Gln Gly Pro Arg Arg Val Asp Lys Arg Thr Leu
       130                 135                 140
   Val Glu Leu His Gly Phe Gln Ala Pro Ala Ala Gln Gly Ala Phe Leu
   145                 150                 155                 160
   Arg Gly Ser Gly Leu Ser Leu Ala Ser Gly Arg Phe Thr Ala Pro Val
                   165                 170                 175
   Ser Gly Ile Phe Gln Phe Ser Ala Ser Leu His Val Asp His Ser Glu
               180                 185                 190
   Leu Gln Gly Lys Ala Arg Leu Arg Ala Arg Asp Val Val Cys Val Leu
           195                 200                 205
   Ile Cys Ile Glu Ser Leu Cys Gln Arg His Thr Cys Leu Glu Ala Val
       210                 215                 220
   Ser Gly Leu Glu Ser Asn Ser Arg Val Phe Thr Leu Gln Val Gln Gly
   225                 230                 235                 240
   Leu Leu Gln Leu Gln Ala Gly Gln Tyr Ala Ser Val Phe Val Asp Asn
                   245                 250                 255
   Gly Ser Gly Ala Val Leu Thr Ile Gln Ala Gly Ser Ser Phe Ser Gly
               260                 265                 270
   Leu Leu Leu Gly Thr
           275
<210> 35
   <211> 849
   <212> DNA
   <213> Homo sapiens
<400> 35
   cggcgggagg cacagaggac gcagcagcct ggccagcgcg cagatccccc caacgccacc    60
   gccagcgcgt cctcccgcga ggggctgccc gaggccccca agccatccca ggcctcagga   120
   cctgagttct ccgacgccca catgacatgg ctgaactttg tccggcggcc ggacgacggc   180
   gccttaagga agcggtgcgg aagcagggac aagaagccgc gggatctctt cggtccccca   240
   ggacctccag gtgcagaagt gaccgcggag actctgcttc acgagtttca ggagctgctg   300
   aaagaggcca cggagcgccg gttctcaggg cttctggacc cgctgctgcc ccagggggcg   360
   ggcctgcggc tggtgggcga ggcctttcac tgccggctgc agggtccccg ccgggtggac   420
   aagcggacgc tggtggagct gcatggtttc caggctcctg ctgcccaagg tgccttcctg   480
   cgaggctccg gtctgagcct ggcctcgggt cggttcacgg cccccgtgtc cggcatcttc   540
   cagttctctg ccagtctgca cgtggaccac agtgagctgc agggcaaggc ccggctgcgg   600
   gcccgggacg tggtgtgtgt tctcatctgt attgagtccc tgtgccagcg ccacacgtgc   660
   ctggaggccg tctcaggcct ggagagcaac agcagggtct tcacgctaca ggtgcagggg   720
   ctgctgcagc tgcaggctgg acagtacgct tctgtgtttg tggacaatgg ctccggggcc   780
   gtcctcacca tccaggcggg ctccagcttc tccgggctgc tcctgggcac gcaccatcac   840
   catcaccat                                                           849
<210> 36
   <211> 283
   <212> PRT
   <213> Homo sapiens
<400> 36
   Arg Arg Glu Ala Gln Arg Thr Gln Gln Pro Gly Gln Arg Ala Asp Pro
   1               5                   10                  15
   Pro Asn Ala Thr Ala Ser Ala Ser Ser Arg Glu Gly Leu Pro Glu Ala
   20 25 30
   Pro Lys Pro Ser Gln Ala Ser Gly Pro Glu Phe Ser Asp Ala His Met
   35 40 45
   Thr Trp Leu Asn Phe Val Arg Arg Pro Asp Asp Gly Ala Leu Arg Lys
   50 55 60
   Arg Cys Gly Ser Arg Asp Lys Lys Pro Arg Asp Leu Phe Gly Pro Pro
   65 70 75 80
   Gly Pro Pro Gly Ala Glu Val Thr Ala Glu Thr Leu Leu His Glu Phe
   85 90 95
   Gln Glu Leu Leu Lys Glu Ala Thr Glu Arg Arg Phe Ser Gly Leu Leu
   100 105 110
   Asp Pro Leu Leu Pro Gln Gly Ala Gly Leu Arg Leu Val Gly Glu Ala
   115 120 125
   Phe His Cys Arg Leu Gln Gly Pro Arg Arg Val Asp Lys Arg Thr Leu
   130 135 140
   Val Glu Leu His Gly Phe Gln Ala Pro Ala Ala Gln Gly Ala Phe Leu
   145 150 155 160
   Arg Gly Ser Gly Leu Ser Leu Ala Ser Gly Arg Phe Thr Ala Pro Val
   165 170 175
   Ser Gly Ile Phe Gln Phe Ser Ala Ser Leu His Val Asp His Ser Glu
   180 185 190
   Leu Gln Gly Lys Ala Arg Leu Arg Ala Arg Asp Val Val Cys Val Leu
   195 200 205
   Ile Cys Ile Glu Ser Leu Cys Gln Arg His Thr Cys Leu Glu Ala Val
   210 215 220
   Ser Gly Leu Glu Ser Asn Ser Arg Val Phe Thr Leu Gln Val Gln Gly
   225 230 235 240
   Leu Leu Gln Leu Gln Ala Gly Gln Tyr Ala Ser Val Phe Val Asp Asn
   245 250 255
   Gly Ser Gly Ala Val Leu Thr Ile Gln Ala Gly Ser Ser Phe Ser Gly
   260 265 270
   Leu Leu Leu Gly Thr His His His His His His
   275 280
<210> 37
   <211> 24
   <212> DNA
   <213> Artificial Sequence
<220>
   <223> Primer INSP163-CP1
<400> 37
   tgagccgcct cgggacggag ccat 24
<210> 38
   <211> 24
   <212> DNA
   <213> Artificial Sequence
<220>
   <223> Primer INSP163-CP2
<400> 38
   acgtgcccag gagcagcccg gaga 24
<210> 39
   <211> 35
   <212> DNA
   <213> Artificial sequence
<220>
   <223> Primer INSP163-EX1
<400> 39
   gcaggcttcg ccaccatgcg gcgctgggcc tgggc 35
<210> 40
   <211> 35
   <212> DNA
   <213> Artificial sequence
<220>
   <223> Primer INSP163-EX2
<400> 40
   tgatggtgat ggtgcgtgcc caggagcagc ccgga 35
<210> 41
   <211> 37
   <212> DNA
   <213> Artificial sequence
<220>
   <223> Primer GCP Forward
<400> 41
   ggggacaagt ttgtacaaaa aagcaggctt cgccacc 37
<210> 42
   <211> 51
   <212> DNA
   <213> Artificial sequence
<220>
   <223> Primer GCP Reverse
<400> 42
   ggggaccact ttgtacaaga aagctgggtt tcaatggtga tggtgatggt g 51
<210> 43
   <211> 20
   <212> DNA
   <213> Artificial sequence
<220>
   <223> Primer pEAK12F
<400> 43
   gccagcttgg cacttgatgt 20
<210> 44
   <211> 20
   <212> DNA
   <213> Artificial sequence
<220>
   <223> Primer pEAK12R
<400> 44
   gatggaggtg gacgtgtcag 20
<210> 45
   <211> 18
   <212> DNA
   <213> Artificial sequence
<220>
   <223> Primer 21M13
<400> 45
   tgtaaaacga cggccagt 18
<210> 46
   <211> 18
   <212> DNA
   <213> Artificial sequence
<220>
   <223> Primer M13REV
<400> 46
   caggaaacag ctatgacc 18
<210> 47
   <211> 19
   <212> DNA
   <213> Artificial sequence
<220>
   <223> Primer T7
<400> 47
   taatacgact cactatagg 19
<210> 48
   <211> 18
   <212> DNA
   <213> Artificial Sequence
<220>
   <223> Primer T3
<400> 48
   attaaccctc actaaagg 18
<210> 49
   <211> 20
   <212> DNA
   <213> Artificial sequence
<220>
   <223> Primer h-INSP163-exon3-4-331F
<400> 49
   GAAGTGACCGCGGAGACTCT 20
<210> 50
   <211> 17
   <212> DNA
   <213> Artificial sequence
<220>
   <223> h-INSP163-exon3-4-392R
<400> 50
   CGCTCCGTGGCCTCTTT 17

## Description

### FIELD OF THE INVENTION

The invention relates to the use of INSP163 for the treatment and/or prevention of lung cancer.

### BACKGROUND OF THE INVENTION

Bronchogenic Carcinoma is a highly malignant primary lung tumor that accounts for most cases of lung cancer and has a very poor prognosis. Bronchogenic carcinoma accounts for > 90% of all lung tumors. It is the second most common cancer in men (13%) and the third most common cancer in women (13%). It is the leading cause of cancer death among men (32%) and women (25%), and its incidence appears to be rising more rapidly among women. It is most common between the ages of 45 and 70.

Four histologic types of bronchogenic carcinoma usually are distinguished: squamous cell, commonly arising in the larger bronchi and spreading by direct extension and lymph node metastasis; undifferentiated small cell, often associated with early hematogenous metastases; undifferentiated large cell, usually spreading through the bloodstream; and adenocarcinoma, commonly peripheral, often spreading through the bloodstream, All types also commonly spread via the lymphatics.

Bronchioloalveolar carcinoma, a subtype of adenocarcinoma, consolidates airspaces and often does not extend beyond the lungs. Although a solitary form exists, this cancer is sometimes distinguished from other types of bronchogenic carcinoma by its multifocal origin.

Horner's syndrome (due to invasion of the cervical thoracic sympathetic nerves) consists of enophthalmos, miosis, ptosis, and ipsilateral facial anhidrosis.

Pancoast syndrome (due to infiltration of the brachial plexus and neighboring ribs and vertebrae) consists of pain, numbness, and weakness of the affected arm. The two syndromes may coexist.

Paraneoplastic syndromes of lung cancer, which are numerous, are extrapulmonary, remote effects of tumors. They lead to metabolic and neuromuscular disturbances unrelated to the primary tumor or metastases. They may be the first sign of occurrence or recurrence, but they do not necessarily indicate that a tumor has spread outside the chest. In hypertrophic pulmonary osteoarthropathy (the best known), clubbing of the fingers and toes and periosteal elevation of the distal parts of long bones occur. All levels of the nervous system may be affected-principally causing encephalopathy, subacute cerebellar degeneration, encephalomyelitis, the Eaton-Lambert syndrome, and peripheral neuropathy. Polymyositis and dermatomyositis or metabolic syndromes due to production of substances with hormonal activity may develop. Small cell carcinomas may secrete ectopic ACTH, resulting in Cushing's syndrome, or ADH, causing water retention and hyponatremia, and are also associated with the carcinoid syndrome (flushing, wheezing, diarrhea, and cardiac valvular lesions). Squamous cell carcinomas may secrete parathyroid hormone-like substances that produce hypercalcemia. Other endocrine syndromes associated with primary lung carcinomas include gynecomastia, hyperglycemia, thyrotoxicosis, and skin pigmentation. Hematologic disorders, including thrombocytopenic purpura, leukemoid reaction, myelophthisic anemia, polycythemia, and marantic thrombosis, may also occur.

Squamous cell carcinoma is a common form of lung cancer, accounting for approximately one-third of all cases of bronchogenic carcinomas. Unlike adenocarcinoma, it is strongly linked with a history of cigarette smoking. Its histogenesis may be related to chronic inflammation and injury of the bronchial epithelium, which leads to replacement of the normal ciliated columnar epithelium by a squamous epithelium. This transformation from a glandular epithelium to squamous epithelium is known as squamous metaplasia.

Bronchogenic carcinoma has a poor prognosis. On average, patients with untreated bronchogenic carcinoma survive 8 months; about 10 to 35% of tumors are resectable, but the overall 5-year survival rate is approximately 13%. In patients with well-circumscribed, slow-growing tumors, the 5-year survival rate after excision ranges from 15% in patients with stage IIIA non-small cell carcinoma to 70% in patients with stage I non-small cell carcinoma. Best results are obtained in patients with peripheral nodular lesions treated by lobectomy. Second primary lung cancers develop in 6 to 12% of survivors. Because small cell carcinoma has almost always spread beyond the primary site at the time of diagnosis, it is usually inoperable. Rarely, early-stage small cell carcinoma can be surgically resected, but because tumors are likely to recur, adjuvant chemotherapy with cisplatin and etoposide is usually recommended. A second primary cancer develops after treatment of early-stage small cell carcinoma in 25 to 50% of cases.

Prevention of bronchogenic carcinoma includes avoiding tobacco and exposure to potentially carcinogenic substances in industry.

Chemotherapy with multiple drugs, particularly cisplatin and topoisomerase inhibitors--with or without radiation therapy-has yielded higher survival rates than surgery has in patients with small cell carcinoma; cures are rare. Chemotherapy in unresectable stage IIIA, IIIB, or IV non-small cell lung carcinomas appears to improve median survival by 6 to 12 weeks on average and can effectively ameliorate symptoms of the disease in patients who respond. Drugs active in this disease include platinum compounds (cisplatin and carboplatin), vinca alkaloids (vinorelbine, vincristine, and vinblastine), taxines (docetaxel and paclitaxel), and various topolsomerase inhibitors.

The protein INSP163 was disclosed in WO 2005/042576 as a secreted protein containing a jelly-roll fold, in particular, as a member of the TNF (tumor necrosis factor)-like family of cytokines, specifically as a c1q-like protein. No experimental data have been provided so far to show any involvement of INSP163 in cancer and/or musculoskeletal/connective tissue disorder.

### SUMMARY OF THE INVENTION

The invention is based on the unexpected finding that INSP163 displays restricted expression in specific tissues, namely in lung tumor and osteoarthiritis tissues.

It is therefore a first object of the invention to use INSP163 for the preparation of a medicament for the treatment and/or prevention of lung cancer. It is a second object of the invention to use a cell expressing INSP163, or an expression vector comprising the coding sequence of INSP163, for the preparation of a medicament for the treatment and/or prevention of lung cancer. The present invention is also directed towards a pharmaceutical composition comprising INSP163 for the treatment and/or prevention of lung cancer.

### DESCRIPTION OF THE INVENTION

The invention is based on the unexpected finding of the restricted expression of INSP163 in one lung tumor tissue and in two osteoarthritis tissues. The lung tumor is a bronchogenic carcinoma, more specifically a squamous cell carcinoma. This specific pattern of expression of INSP163 leads to the conclusion of the involvement of INSP163 in lung cancer and/or osteoarthritis. These surprising properties presently characterized of the polynucleotides or the corresponding polypeptides of WO 2005/042576 make them particularly suitable for the preparation of a medicament or pharmaceutical composition.

In a first aspect, the invention therefore relates to the use of a polypeptide for the preparation of a medicament for the treatment and/or prevention of lung cancer, wherein said polypeptide is selected from the group consisting of:
a) A polypeptide consisting of SEQ ID NO: 30, or
b) A polypeptide comprising any of SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 30, or SEQ ID NO: 34 or
c) A glycosylated form of the polypeptide, wherein the polypeptide is glycosylated at one or more sites, or
d) A salt or fusion protein, of any of (a) to (c).

In a second aspect, the invention relates to the use of a nucleic acid molecule for the preparation of a medicament for the treatment and/or prevention of a lung cancer, wherein said nucleic acid is selected from the group consisting of:
a) A nucleic acid sequence as set forth in any of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 29 or SEQ ID NO: 33 or
b) A nucleic acid sequence of (a) wherein said nucleic acid sequence encodes an amino acid sequence having conservative amino acid substitutions to the amino acid sequences in SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 30 or SEQ ID NO: 34.

In a third aspect, the invention relates to a pharmaceutical composition for the treatment and/or prevention of lung cancer comprising a polypeptide selected from the group consisting of:
a) A polypeptide consisting of SEQ ID NO: 30, or
b) A polypeptide comprising any of SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 30 or SEQ ID NO: 34 or
c) A glycosylated form of the polypeptide, wherein the polypeptide is glycosylated at one or more sites, or
d) A salt or fusion protein of any of (a) to (c).

In a fourth aspect, the invention relates to a pharmaceutical composition for the treatment and/or prevention of a lung cancer comprising a nucleic acid selected from the group consisting of:
a) A nucleic acid sequence as set forth in any of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 29 or SEQ ID NO: 33 or
b) A nucleic acid sequence of (a) wherein said nucleic acid sequence encodes an amino acid sequence having conservative amino acid substitutions to the amino acid sequences in SEQ ID NO: 2, SEQ ID NO: 4, SEQ ID NO: 6, SEQ ID NO: 8, SEQ ID NO: 10, SEQ ID NO: 12, SEQ ID NO: 14 SEQ ID NO: 30 or SEQ ID NO: 34.

It will be appreciated by the person skilled in the art that in accordance with the present invention, a substance which simulates release or potentiates the activity of endogenous INSP163 can equally be used for treatment and/or prevention of lung cancer.

The polypeptide having the sequence recited in SEQ ID NO: 2 is referred to hereafter as the "alternative mature INSP163 polypeptide". The polypeptide having the sequence recited in SEQ ID NO: 34 is referred to hereafter as the "mature INSP163 polypeptide". SEQ ID NO: 2 or SEQ ID NO: 34 refer herein to a "mature form". The polypeptide having the sequence recited in SEQ ID NO: 4 is referred to hereafter as the "INSP163-A polypeptide". The polypeptide having the sequence recited in SEQ ID NO:6 is referred to hereafter as the "INSP163-B polypeptide". The polypeptide having the sequence recited in SEQ ID NO: 8 is referred to hereafter as the "INSP163-C polypeptide". The polypeptide having the sequence recited in SEQ ID NO: 10 is referred to hereafter as the "INSP183-D polypeptide". The polypeptide having the sequence recited in SEQ ID NO: 12 is referred to hereafter as the "INSP163-E polypeptide". The polypeptide having the sequence recited in SEQ ID NO: 14 is referred to hereafter as the "INSP163-F polypeptide". SEQ ID NO: 4, SEQ ID NO: 6, SEQ ID NO: 8, SEQ ID NO: 10, SEQ ID NO: 12, or SEQ ID NO: 14 refer herein to a "cleaved form".

Although the Applicant does not wish to be bound by this theory, it is postulated that the INSP163 polypeptide further comprises a signal peptide at the N-terminus that is 25 amino acids in length leading to a mature INSP163 polypeptide.

Furthermore, it is postulated that the INSP163 polypeptide further comprises a signal peptide at the N-terminus that is 20 amino acids in length leading to an alternative mature INSP163 polypeptides.

The mature INSP163 polypeptide and alternative mature INSP163 polypeptide sequence with their respective postulated signal sequence is recited in SEQ ID NO: 30.

The polypeptide having the sequence recited in SEQ ID NO: 30 is referred to hereafter as "the INSP163 polypeptide."

The polypeptides disclosed herein may further comprise a histidine tag and are herein referred to as a "histidine tag form". Preferably the histidine tag is found at the C-terminus of the polypeptide. Preferably the histidine tag comprises 1-10 histidine residues (e.g. 1, 2, 3, 4, 5, 6, 7, 8, 9 or 10 residues). More preferably, the histidine tag comprises 6 residues. Preferred polypeptides are therefore those comprising the sequence recited in SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 32 and/or SEQ ID NO: 36.

The polypeptide having the sequence recited in SEQ ID NO: 16 is referred to hereafter as the "histidine tag alternative mature INSP163 polypeptide". The polypeptide having the sequence recited in SEQ ID NO: 18 is referred to hereafter as the "histidine tag lNSP163-A polypeptide". The polypeptide having the sequence recited in SEQ ID NO: 20 is referred to hereafter as the "histidine tag INSP163-B polypeptide". The polypeptide having the sequence recited in SEQ ID NO: 22 is referred to hereafter as the "histidine tag INSP163-C polypeptide". The polypeptide having the sequence recited in SEQ ID NO: 24 is referred to hereafter as the "histidine tag INSP163-D polypeptide". The polypeptide having the sequence recited in SEQ ID NO: 26 is referred to hereafter as the "histidine tag INSP183-E polypeptide". The polypeptide having the sequence recited in SEQ ID NO: 28 is referred to hereafter as the "histidine tag INSP163-F polypeptides. The polypeptide having the sequence recited in SEQ ID NO: 32 is referred to hereafter as the "histidine tag INSP163 polypeptides". The polypeptide having the sequence recited in SEQ ID NO: 36 is referred to hereafter as the "histidine tag mature INSP163 polypeptide".

The term INSP163 polypeptides" or "INSP163" as used herein includes the alternative mature INSP163 polypeptide, the mature INSP163 polypeptides, the INSP-163-A polypeptide, the INSP163-B polypeptide, the INSP163-C polypeptide, the INSP163-D polypeptide, the INSP163-E polypeptide, the INSP163-F polypeptide, the INSP163 polypeptide, the histidine tag alternative mature INSP163 polypeptide, the histidine tag INSP163-A polypeptide, the histidine tag INSP163-B polypeptide, the histidine tag INSP163-C polypeptide, the histidine tag INSP163-D polypeptide, the histidine tag INSP163-E polypeptide, the histidine tag INSP163-F polypeptide, the histidine tag INSP163 polypeptide, the histidine tag mature INSP163 polypeptide and the full length INSP163 (mature INSP163 polypeptide with signal peptide).

The nucleic acid molecule described herein comprises the nucleic acid sequence as recited in SEQ ID NO: 1 (encoding the alternative nature INSP163 polypeptide), SEQ ID NO: 3 (encoding the INSP163-A polypeptide), SEQ ID NO: 5 (encoding the INSP163-B polypeptide), SEQ ID NO: 7 (encoding the INSP163-C polypeptide), SEQ ID NO: 9 (encoding the INSP163-D polypeptide), SEQ ID NO: 11 (encoding the INSP163-E polypeptide), SEQ ID NO: 13 (encoding the INSP163-F polypeptide), SEQ ID NO: 15 (encoding the histidine tag alternative mature INSP163 polypeptide), SEQ ID NO: 17 (encoding the histidine tag INSP163-A polypeptide), SEQ ID NO: 19 (encoding the histidine tag INSP163-B polypeptide), SEQ ID NO: 21 (encoding the histidine tag INSP163-C polypeptide), SEQ ID NO: 23 (encoding the histidine tag INSP163-D polypeptide), SEQ ID NO: 25 (encoding the histidine tag INSP163-E polypeptide), SEQ ID NO: 27 (encoding the histidine tag INSP163-F polypeptide), SEQ ID NO: 29 (encoding the INSP163 polypeptide), SEQ ID NO: 31 (encoding the histidine tag INSP163 polypeptide), SEQ ID NO: 33 (encoding the mature INSP163 polypeptide), SEQ ID NO: 35 (encoding the histidine tag mature INSP163 polypeptide).

The nucleic acid molecule herein disclosed consists of the nucleic acid sequence recited in SEQ ID NO: 1 (encoding the alternative mature INSP163 polypeptide), SEQ ID NO: 3 (encoding the INSP163-A polypeptide), SEQ ID NO: 5 (encoding the INSP163-B polypeptide), SEQ ID NO: 7 (encoding the INSP163-C polypeptide), SEQ ID NO: 9 (encoding the INSP163-D polypeptide), SEQ ID NO: 11 (encoding the INSP163-E polypeptide), SEQ ID NO: 13 (encoding the INSP163-F polypeptide), SEQ ID NO: 15 (encoding the histidine tag alternative mature INSP163 polypeptide), SEQ ID NO: 17 (encoding the histidine tag INSP163-A polypeptide), SEQ ID NO: 19 (encoding the histidine tag INSP163-B polypeptide), SEQ ID NO: 21 (encoding the histidine tag INSP163-C polypeptide), SEQ ID NO: 23 (encoding the histidine tag INSP163-D polypeptide), SEQ ID NO: 25 (encoding the histidine tag INSP163-E polypeptide), SEQ ID NO: 27 (encoding the histidine tag INSP163-F polypeptide), SEQ ID NO: 29 (encoding the INSP163 polypeptide), SEQ ID NO: 31 (encoding the histidine tag INSP163 polypeptide), SEQ ID NO: 33 (encoding the mature INSP163 polypeptide), SEQ ID NO: 35 (encoding the histidine tag mature INSP163 polypeptide).

The term "treatment and/or prevention" as used herein encompasses any attenuation, reduction, or partial, substantial or complete prevention or blockage of disease formation, development, progression or of the formation, development or progression of any one or several or all of the symptoms of the disease.

Preferably, "tumors of the lungs" or "lung cancer", as used interchangeably herein, are selected from benign or malignant primary tumors or from metastases from primary cancers of many other organs and tissues.

Preferably the lung cancer is selected from primary lung tumors including bronchogenic carcinoma, bronchial carcinoid, chondromatous hamartoma (benign), solitary lymphoma, sarcoma (malignant) or multifocal lymphomas.

Preferably, the bronchogenic carcinoma is selected from squamous cell carcinoma, undifferentiated small cell carcinoma, undifferentiated large cell carcinoma, adenocarcinoma or Bronchioloalveolar carcinoma.

Preferably, the bronchogenic carcinoma is squamous cell carcinoma or non-small cell lung carcinoma.

Preferably the lung cancer is selected from metastases from primary cancers of the skin, breast, colon, prostate, kidney, thyroid, stomach, cervix, rectum, testis, and bone and from melanorna.

"Bronchial carcinoid" or "bronchial adenoma", terms that can be used interchangeably, may be benign or malignant and occurs equally in both sexes. Its course is prolonged. The endobronchial portion of the tumor may obstruct the lumen of major bronchi. Brisk bleeding from the overlying mucous membrane often occurs. Recurrent pneumonia within the same lung zone and localized overlying pleural pain are common. Metastases are uncommon but may occur to regional lymph nodes. prolonged overuse of a joint or group of joints associated with occupations such as foundry work, coal mining, and bus driving).

The term "INSP163" as used herein, relates to a protein comprising SEQ ID NO: 2, SEQ ID NO: 4, SEQ ID NO: 6, SEQ ID NO: 8, SEQ ID NO: 10, SEQ ID NO:12, SEQ ID NO: 14, SEQ ID NO: 30 or SEQ ID NO: 34 (all human) of the enclosed sequence listing, as well as to salts, thereof. INSP163 from species other than human, such as mouse or rat, may be used in accordance with the present invention, as long as there is a sufficient identity between the proteins as to allow the protein to exhibit its biological activity, and without eliciting a substantial immune response in a human being.

The term "INSP163", as used herein, relates to SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID

NO: 30 or SEQ ID NO: 34 showing the desired activity in lung cancer Differentially glycosylated or sialylated forms of the protein may be used according to the invention, as long as they exhibit any beneficial effect on cancer and/or musculoskeletal/connective tissue disorder, preferably an effect which is at least comparable of the full length protein. The beneficial effect can be measured in one of the *in vitro* or *in vivo* tests described in the examples below, or in any other assay adequate to demonstrate an effect in lung cancer.

In accordance with the present invention, INSF163 can be a naturally occurring, i.e. native protein, or a recombinant protein. Recombinant production may be carried out in eukaryotic cells, such as yeast cells or mammalian cells, preferably in CHO cells, HEK cells (human embryonic kidney cells) or in human fibroblast cells or cell lines. It may further be produced in prokaryotic cells such as E. coli.

Preferably, INSP163 is glycosylated at one or more sites. It may also be unglycosylated, depending on the given needs and the source of production or isolation of the protein.

Preferably, the polypeptides of the invention are glycosylated at residues 43 and/or 281 of SEQ ID NO: 30.

The term "salts" herein refers to both salts of carboxyl groups and to acid addition salts of amino groups of INSP163 molecule or analogs thereof. Salts of a carboxyl group may be formed by means known in the art and include inorganic salts, for example, sodium, calcium, ammonium, ferric or zinc salts, and the like, and salts with organic bases as those formed, for example, with amines, such as triethanolamine, arginine or lysine, piperidine, procaine and the like. Acid addition salts include, for example, salts with mineral acids, such as, for example, hydrochloric acid or sulfuric acid, and salts with organic acids, such as, for example, acetic acid or oxalic acid. Of course, any such salts must retain the biological activity of INSP163 relevant to the present invention, i.e., exert a beneficial effect on cancer and/or musculoskeletal/connective tissue disorder, in particular lung cancer and/or osteoarthritis.

Isoforms or splice variants of INSP163 are disclosed herein, as long as they are capable of inhibiting disease progression and/or symptoms of that disease.

The term "muteins" refers to analogs of INSP163. In which one or more of the amino acid residues of natural INSP163 are replaced by different amino acid residues, or are deleted, or one or more amino add residues are added to the natural sequence of INSP163, having preferably at least the same activity as wild type INSP163 or even having a much more potent activity. The biological activity of INSP163 can e.g. be measured by assaying INSP163 in its capacity to induce apoptosis. The assay as described in Example 3 for determining IKK2 activity in non-small cell lung carcinoma cells is suitable to determine if INSP163 can induce cell death. Assays for assessing protein-protein interactions are well known by the person skilled in the art. Examples for such assays are ELISA type binding assays, immuno-precipitation assays, or measurement in any other suitable system such as the BIAcore system. These muteins are prepared by known synthesis and/or by site-directed mutagenesis techniques, or any other known technique suitable therefor.

Any such mutein preferably has a sequence of amino acids sufficiently duplicative of that INSP163, such as to have at least a substantially similar activity of INSP163. The activity of an INSP163 mutant can further be tested in the assays explained in the example below (example 3). Measuring cell death of non-small cell lung carcinoma cells treated with INSP163 may be a suitable test for assessing the activity of INSP163 muteins, for example.

Muteins include proteins encoded by a nucleic acid, such as DNA or RNA, which hybridizes to DNA or RNA, which encodes INSP163 under stringent conditions. The term "stringent conditions" refers to hybridization and subsequent washing conditions, which those of ordinary skill in the art conventionally refer to as "stringent". See Ausubel et al., Current Protocols in Molecular Biology, supra, Interscience, N.Y., §§6.3 and 6.4 (1987, 1992), and Sambrook *et al*. (Sambrook, J. C., Fritsch, E. F., and Maniatis, T. (1989) Molecular Cloning: A Laboratory Manual, Cold Spring Harbor Laboratory Press, Cold Spring Harbor, NY).

Without limitation, examples of stringent conditions include washing conditions 12-20°C below the calculated Tm of the hybrid under study in, *e.g*., 2 x SSC and 0.5% SDS for 5 minutes, 2 x SSC and 0.1% SDS for 15 minutes; 0.1 x SSC and 0.5% SDS at 37°C for 30-60 minutes and then, a 0.9 x SSC and 0.5% SDS at 68°C for 30-60 minutes. Those of ordinary skill in this art understand that stringency conditions also depend on the length of the DNA sequences, oligonucleotide probes (such as 10-40 bases) or mixed oligonucleotide probes. If mixed probes are used, It is preferable to use tetramethyl ammonium chloride (TMAC) instead of SSC. See Ausubel, supra.

Any such mutein preferably has a sequence of amino acids sufficiently duplicative of that of INSP163, such as to have substantially similar, or even better, biological activity as INSP163.

One easily measurable activity of INSP163 is its capability of inducing cell death of cancer cells (e.g. A549 cells). As long as the mutein has substantial activity in inducing cell death of cancer cells (e.g. A549 cells, see Example 3), it can be considered to have substantially similar activity to INSP163. Thus, it can be determined whether any given mutein has at least substantially the same activity as INSP163 by means of routine experimentation comprising subjecting such a mutein.

Any such mutein has at least 40% identity or homology with the sequence of INSP163. More preferably, it has at least 50%, at least 60%, at least 70%, at least 80% or, most preferably, at least 90% identity or homology thereto.

identity reflects a relationship between two or more polypeptide sequences or two or more polynucleotide sequences, determined by comparing the sequences. In general, identity refers to an exact nucleotide to nucleotide or amino acid to amino acid correspondence of the two polynucleotides or two polypeptide sequences, respectively, over the length of the sequences being compared.

For sequences where there is not an exact correspondence, a "% identity" may be determined. In general, the two sequences to be compared are aligned to give a maximum correlation between the sequences. This may include inserting "gaps" in either one or both sequences, to enhance the degree of alignment. A % identity may be determined over the whole length of each of the sequences being compared (so-called global alignment), that is particularly suitable for sequences of the same or very similar length, or over shorter, defined lengths (so-called local alignment), that is more suitable for sequences of unequal length.

Methods for comparing the identity and homology of two or more sequences are well known in the art. Thus for instance, programs available in the Wisconsin Sequence Analysis Package, version 9.1 (Devereux J *et al*, 1984), for example the programs BESTFIT and GAP, may be used to determine the % identity between two polynucleotides and the % identity and the % homology between two polypeptide sequences. BESTFIT uses the "local homology" algorithm of Smith and Waterman (1981) and finds the best single region of similarity between two sequences. Other programs for determining identity and/or similarity between sequences are also known in the art, for instance the BLAST family of programs (Altschul S F *et al,* 1990, Altschul S F *et al*, 1997, accessible through the home page of the NCBI at www.ncbi.nlm.nih.gov) and FASTA (Pearson W R, 1990; Pearson 1988).

Muteins of INSP163, or nucleic acids encoding them, include a finite set of substantially corresponding sequences as substitution peptides or polynucleotides which can be routinely obtained by one of ordinary skill in the art, without undue experimentation, based on the teachings and guidance presented herein.

Preferred changes for muteins are what are known as "conservative" substitutions. Conservative amino acid substitutions of INSP163 polypeptides or proteins, may include synonymous amino acids within a group which have sufficiently similar physicochemical properties that substitution between members of the group will preserve the biological function of the molecule (Grantham, 1974). It is clear that insertions and deletions of amino acids may also be made in the above-defined sequences without altering their function, particularly if the insertions or deletions only involve a few amino acids, e.g., under thirty, and preferably under ten, and do not remove or displace amino acids which are critical to a functional conformation, e.g., cysteine residues.

Preferably, the synonymous amino acid groups are those defined in Table I. More preferably, the synonymous amino acid groups are those defined in Table II; and most preferably the synonymous amino acid groups are those defined in Table III.

**TABLE I**

| Preferred Groups of Synonymous Amino Acids | |
|---|---|
| Amino Acid | Synonymous Group |
| Ser | Ser, Thr, Gly, Asn |
| Arg | Arg, Gln, Lys, Glu, His |
| Leu | Ile, Phe, Tyr, Met, Val, Leu |
| Pro | Gly, Ala, Thr, Pro |
| Thr | Pro, Ser, Ala, Gly, His, Gln, Thr |
| Ala | Gly, Thr, Pro, Ala |
| Val | Met, Tyr, Phe, Ile, Leu, Val |
| Gly | Ala, Thr, Pro, Ser, Gly |
| Ile | Met, Tyr, Phe, Val, Leu, Ile |
| Phe | Trp, Met, Tyr, Ile, Val, Leu, Phe |
| Tyr | Trp, Met, Phe, Ile, Val, Leu, Tyr |
| Cys | Ser, Thr, Cys |
| His | Glu, Lys, Gin, Thr, Arg, His |
| Gln | Glu, Lys, Asn, His, Thr, Arg, Gin |
| Asn | Gln, Asp, Ser, Asn, |
| Lys | Glu, Gln, His, Arg, Lys |
| Asp | Glu, Asn, Asp |
| Glu | Asp, Lys, Asn, Gin, His, Arg, Glu |
| Met | Phe, Ile, Val, Leu, Met |
| Trp | Trp |

**TABLE II**

| More Preferred Groups of Synonymous us Amino Acids | |
|---|---|
| Amino Acid | Synonymous Group |
| Ser | Ser |
| Arg | His, Lys, Arg |
| Leu | Leu, Ile, Phe, Met |
| Pro | Ala, Pro |
| Thr | Thr |
| Ala | Pro, Ala |
| Val | Val, Met, Ile |
| Gly | Gly |
| Ile | Ile, Met, Phe, Val, Leu |
| Phe | Met, Tyr, Ile, Leu, Phe |
| Tyr | Phe, Tyr |
| Cys | Cys, Ser |
| His | His, Gln, Arg |
| Gln | Glu, Gln, His |
| Asn | Asp, Asn |
| Lys | Lys, Arg |
| Asp | Asp, Asn |
| Glu | Glu, Gln |
| Met | Met, Phe, Ile, Val, Leu |
| Trp | Trp |

**TABLE III**

| Most Preferred Groups of Synonymous Amino Acids | |
|---|---|
| Amino Acid | Synonymous Group |
| Ser | Ser |
| Arg | Arg |
| Leu | Leu, Ile, Met |
| Pro | Pro |
| Thr | Thr |
| Ala | Ala |
| Val | Val |
| Gly | Gly |
| Ile | Ile, Met, Leu |
| Phe | Phe |
| Tyr | Tyr |
| Cys | Cys, Ser |
| His | His |
| Gln | Gln |
| Asn | Asn |
| Lys | Lys |
| Asp | Asp |
| Glu | Glu |
| Met | Met, Ile, Leu |
| Trp | Met |

Examples of production of amino acid substitutions in proteins which can be used for obtaining muteins of INSP163 polypeptides or proteins include any known method steps, such as presented in US patents 4,959,314, 4,588,585 and 4,737,462, to Mark et al; 5,116,943 to Koths et al.*,* 4,965,195 to Namen et al*;* 4,879,111 to Chong et al; and 5,017,691 to Lee et al; and lysine substituted proteins presented in US patent No. 4,904,584 (Shaw et al).

The term "fusion protein" refers to a polypeptide comprising INSP163, or a mutein thereof, fused with another protein, which, e.g., has an extended residence time in body fluids. Fusion proteins comprising all or a functional part of INSP163 fused to all or a functional part of a protein capable of improving the biological activities of the molecule, like half-life in the human body, for instance, are preferred according to the invention. In a preferred embodiment the fusion protein comprises an immunoglobulin (Ig) fusion. Fusion proteins comprising all or part of INSP163 fused to all or part of an immunoglobulin are highly preferred. They can be monomeric or multimeric, hetero- or homomultimeric. Advantageously, the fusion protein comprises the constant region of an immunoglobulin, in particular of the Fc portion of the immunoglobulin. Embodiments in which the immunoglobulin is of the IgG1 or IgG2 isotype are further preferred according to the invention. Preferably, the fusion is an Fc fusion.

INSP163 may thus be fused to another protein, polypeptide or the like, e.g., an immunoglobulin or a fragment thereof. The fusion may be direct, or via a short linker peptide which can be as short as 1 to 3 amino acid residues in length or longer, for example, 13 amino acid residues in length. Said linker may be a tripeptide of the sequence E-F-M (Glu-Phe-Met), for example, or a 13-amino acid linker sequence comprising Glu-Phe-Gly-Ala-Gly-Lou-Val-Leu-Gly-Gly-Gln-Phe-Met introduced between the INSP163 sequence and the immunoglobulin sequence.

"Functional derivatives" cover derivatives of INSP163, and their muteins and fusion proteins, which may be prepared from the functional groups which occur as side chains on the residues or the N- or C-terminal groups, by means known in the art, and are disclosed as long as they remain pharmaceutically acceptable, i.e. they do not destroy the activity of the protein which is at least substantially similar to the activity of INSP163, and do not confer toxic properties on compositions containing it. The functional derivative comprises at least one moiety attached to one or more functional groups, which occur as one or more side chains on the amino acid residues.

Polyethylene glycol (PEG) side-chains are highly preferred moieties. PEG side chains may mask antigenic sites and extend the residence of the substance they are attached to in body fluids. Other derivatives include aliphatic esters of the carboxyl groups, amides of the carboxyl groups by reaction with ammonia or with primary or secondary amines, N-acyl derivatives of free amino groups of the amino acid residues formed with acyl moieties (e.g. alkanoyl or carbocyclic aroyl groups) or O-acyl derivatives of free hydroxyl groups (for example that of seryl or threonyl residues) formed with acyl moieties.

"Active fractions" of INSP163 and its muteins and fusion proteins, cover any fragment or precursors of the polypeptide chain of the protein molecule alone or together with associated molecules or residues linked thereto, e.g., sugar or phosphate residues, or aggregates of the protein molecule or the sugar residues by themselves, provided said active fraction has at least a substantially similar activity to INSP163.

In accordance with the present invention, INSP163 may also be administered to the human body In form of a vector comprising said nucleic acid molecule. Therefore, the invention further relates to the use of a vector comprising said nucleic acid molecule for the manufacture of a medicament for the treatment and/or prevention of lung cancer. Preferably, the vector is an expression vector, comprising a promoter operably linked to all or part of the coding sequence of INSP163. In a further preferred embodiment, the vector is a gene therapy vector. Gene therapy vectors are known in the art, most of them are virally derived vectors, such as adenoviral or lentiviral vectors.

According to the invention, INSP163 may also be administered to the human body in form of a cell producing and/or secreting INSP163. Therefore, the invention further relates to the use of a cell expressing INSP163 for the manufacture of a medicament for the treatment and/or prevention of lung cancer i.e. to cell therapy for the treatment and/or prevention of lung cancer. The cell may be a naturally producing INSP163 and/or a transfected cell that produces recombinant INSP163. Preferred are cells expressing and secreting high amounts of the protein, such as over-expressing cells carrying high copy numbers of an expression vector comprising a nucleic acid molecule encoding INSP163.

The invention further relates to a cell comprising a vector comprising a nucleic acid molecule encoding all or part of INSP163 for the preparation of a medicament for treatment and/or prevention of lung cancer. A cell that has been genetically modified to produce a polypeptide according to the invention is also within the scope of the present invention.

The use of an expression vector for inducing and/or enhancing the endogenous production of INSP163 in a cell normally silent or expressing amounts of the inhibitor which are not sufficient, are also contemplated according to the invention. Thus, the invention makes use of a technology known as endogenous gene activation (EGA) for the production of the desired protein.

According to the invention, INSP163 can be administered alone or in combination with several other therapeutic regimens or agents (e.g. multiple drug regimens) to obtain an additive or synergistic effect for the treatment and/or prevention of lung cancer.

Therefore, preferably, the medicament of the invention further comprises:
- An anti-cancer agent

The anti-cancer agent is selected from platinum compounds such as cisplatin and carboplatin, vinca alkaloids such as vinorelbine, vincristine, and vinblastine, taxines such as docetaxel and paclitaxel, and various topolsomerase inhibitors.

All treatments are intended for simultaneous, sequential or separate use.

Pharmaceutical compositions comprising one or more of the above substances, together with INSP163, are within the scope of the present invention.

Also disclosed is a pharmaceutical composition comprising INSP163, optionally together with one or more pharmaceutically acceptable carriers, diluents or excipients, for the treatment and/or prevention of lung cancer. The pharmaceutical composition may further comprise any of the above-identified further components.

The pharmaceutical composition according to the invention may also comprise a vector comprising a nucleic acid molecule according to the Invention, or a cell expressing INSP163.

The active ingredients of the pharmaceutical, i.e. polypeptides, nucleic acids or cells according to the invention, or combinations thereof, as well as the combinations of substances mentioned above, may be administered to an individual in a variety of ways. The routes of administration include intradermal, transdermal (e.g. in slow release formulations), intramuscular, intraperitoneal, Intravenous, subcutaneous, oral, epidural, topical, and intranasal routes. Any other therapeutically efficacious route of administration can be used, for example absorption through epithelial or endothelial tissues or by gene therapy wherein a DNA molecule encoding the active agent is administered to the patient (e.g. via a vector), which causes the active agent to be expressed and secreted in vivo. In addition, the protein(s) according to the invention can be administered together with other components of biologically active agents such as pharmaceutically acceptable surfactants, excipients, carriers, diluents and vehicles.

The definition of "pharmaceutically acceptable" is meant to encompass any carrier, which does not interfere with effectiveness of the biological activity of the active ingredient and that is not toxic to the host to which it is administered. For example, for parenteral administration, the active protein(s) may be formulated in a unit dosage form for injection in vehicles such as saline, dextrose solution, serum albumin and Ringer's solution.

For parenteral (e.g. intravenous, subcutaneous, intramuscular) administration, the active protein(s) can be formulated as a solution, suspension, emulsion or lyophilised powder in association with a pharmaceutically acceptable parenteral vehicle (e.g. water, saline, dextrose solution) and additives that maintain isotonicity (e.g. mannitol) or chemical stability (e.g. preservatives and buffers). The formulation is sterilized by commonly used techniques.

The bioavailability of the active protein(s) can also be ameliorated by using conjugation procedures which increase the half-life of the molecule in the human body, for example linking the molecule to polyethylenglycol, as described in the PCT Patent Application WO 92/13095.

The therapeutically effective amount of the active protein(s) will be a function of many variables, including the type of receptor, the affinity of the substance according to the invention to its receptor, any residual cytotoxic activity exhibited thereby, the route of administration, the clinical condition of the patient.

A "therapeutically effective amount" is such that when administered, the substance according to the invention results in a beneficial effect on disease development or progression *in vivo.* The dosage administered, as single or multiple doses, to an individual will vary depending upon a variety of factors, including the pharmacokinetic properties of INSP163, the route of administration, patient conditions and characteristics (sex, age, body weight, health, size), extent of symptoms, concurrent treatments, frequency of treatment and the effect desired. Adjustment and manipulation of established dosage ranges are well within the ability of those skilled in the art.

The dose of the polypeptide will vary from about 0,0001 to 100 mg/kg or about 0.01 to 10 mg/kg or about 0.1 to 5 mg/kg or about 1 to 3 mg/kg, although as noted above this will be subject to a great deal of therapeutic discretion. The medicament of the invention may be administered daily, every other day, or three times per week.

The daily doses are usually given in divided doses or in sustained release form effective to obtain the desired results. Second or subsequent administrations can be performed at a dosage, which is the same, less than or greater than the initial or previous dose administered to the individual. A second or subsequent administration can be administered during or prior to onset of the disease.

The expression vector may be administered systemically. Preferably the expression vector is administered by intramuscular injection. A further preferred route of administration is inhalation, in particular if lung cancer is involved in the disease. Topical administration of an expression vector comprising INSP163 sequences, or of an INSP163 polypeptide according to the invention, is a further preferred route of administration, in particular if there is an involvement of the skin.

A method for the preparation of a pharmaceutical composition comprises admixing an effective amount of INSP163 with a pharmaceutically acceptable carrier.

Reference to known method steps, conventional methods steps, known methods or conventional methods is not any way an admission that any aspect, description or embodiment of the present invention is disclosed, taught or suggested in the relevant art.

The foregoing description of the specific embodiments will so fully reveal the general nature of the invention that others can, by applying knowledge within the skill of the art (including the contents of the references cited herein), readily modify and/or adapt for various application such specific embodiments, without undue experimentation, without departing from the general concept of the present invention. Therefore, such adaptations and modifications are intended to be within the meaning an range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

Having now described the invention, it will be more readily understood by reference to the following examples that are provided by way of illustration and are not intended to be limiting of the present invention.

### EXAMPLES

### Example 1 - Cloning and expression

Cloning of INSP163 and construction of mammalian cell expression vectors for INSP163 are described in Example 4 and Example 5 of International Application No. PCT/GB2004/004544.

### Example 2 - Analysis of INSP163 gene expression levels by TaqMan analysis

Total RNA from each sample was reverse transcribed using the Superscript III First-Strand Synthesis System for RT-PCR (Invitrogen, Cat. No. 18080-051) in a final reaction volume of 20 µl. 2 µg of total RNA was combined with 50 ng random hexamer primers. 10mM each of dATP, dGTP, dCTP, & dTTP, and DEPC-treated water in a volume of 10 µl. The mixture was incubated at 65 °C for 5 min then chilled on ice for 1 min. The following 10 µl cDNA synthesis mix was prepared in a separate tube: 2 µl 10X RT buffer, 4 µl 25mM MgCl₂, 2 µl 0.1 M DTT, 1 µl RnaseOUT™ (40 units/µl), and 1 µl SuperScript™ III RT enzyme (200 units/µl). The cDNA synthesis mix was added to the RNA/primer mixture, mixed gently and incubated at 25 °C for 10 min then at 50 °C for 50 min. The RT enzyme was then inactivated by incubating at 85 °C for 5 min. The mixture was chilled on ice and then 1 µl of *E. coli* Rnase H (2 units/µl) was added and the mixture incubated at 37 °C for 20 min. The mixture was chilled on ice and then diluted 1/250 with sterile water. Dilutions of the reverse transcriptase reaction were then subjected to real time PCR analysis on a TaqMan instrument (PE Biosystems 7700).

PCR primers for human INSP163 and the housekeeping control gene glyceraldehyde 3-phosphate dehydrogenase (GAPDH) were designed using the Primer Express software (PE Biosystems). The primers selected were h-INSP163-exon3-4-331 F (SEQ ID NO: 49) and h-INSP163-exon3-4-392R (SEQ ID NO: 50). The specificity and the optimal primer concentration to use for the TaqMan analysis were determined by testing the INSP163 gene-specific primers on a series of dilutions of plasmid pCR4-TOPO-INSP163. Potential genomic DNA contamination of the cDNA was excluded by performing PCR reactions using primers specific for GAPDH intronic sequence. The absence of non-specific amplification was controlled by analyzing the PCR products on 4% agarose gels to ensure a single band of the expected molecular weight was produced.

SYBR Green Real-Time PCR reactions were carried out in a reaction volume of 50 µl containing 25 µl SYBR Green PCR master mix (PE Biosystems) (to which 0.5 units AmpErase Uracil N-Glycosylase (UNG, PE Biosystems) had previously been added), 300 nM of each amplification primer, and 5 µl of RT-PCR product. Cycling was performed using the ABI PRISM 7700 (TaqMan) Detection System programmed as follows: 1 cycle of 50 °C for 2 min; 1 cycle of 95 °C for 10 min; 40 cycles of 95 °C for 15 sec, 60 °C for 1 min. Each reaction was carried out in duplicate and the results averaged.

The primer-specific regions of the reverse-transcribed cDNA samples were thus amplified and their cycle threshold (Ct) values determined. The Ct value for each cDNA sample was normalized to that of the housekeeping gene GAPDH as follows. The difference in expression level between the GAPDH gene and the INSP163 gene in each cDNA sample was expressed as a difference in Ct value, i.e. Delta (δ) Ct = Ct (GAPDH) - Ct (INSP163). Results for each sample were then expressed as a fold difference in the number of cycles required for detectable INSP163 gene expression relative to that for GAPDH, according to the formula Fold Difference = 2^{(-δCt)}. Finally, the expression level of the INSP163 gene in each cDNA sample was shown relative to the GAPDH gene expression level, where GAPDH expression level = 100%, by dividing 100 by the Fold Difference for INSP163. Results are shown in table 4.

**Table 4. Expression of INSP163 in various human tissues as measured by RT-PCR (TaqMan).**

| CDNA | EXPRESSION RELATIVE TO 3APDH (=100) |
|---|---|
| S76 Brain | 0.05 |
| S77 Heart | 0.07 |
| S78 Kidney | 0.29 |
| 379 liver | 0.00 |
| S80 Lung | 0.08 |
| S81 Placenta | 0.11 |
| S82 skeletal Muscle | 0.00 |
| S83 small intestine | 0.20 |
| S84 Spleen | 0.12 |
| S85 Thymus | 0.03 |
| S86 Uterus | 0.16 |
| S87 Bone Marrow | 0.03 |
| S88 Thyroid | 0.04 |
| S89 Spinal cord | 0.07 |
| S 90 Cervix | 0.27 |
| S91 colon | 0.03 |
| S92 ovary | 0.04 |
| S93 prostate | 0.03 |
| S94 testis | 0.07 |
| S95 skin | 0.50 |
| S113 pancreas | 0.28 |
| S115 Salivary gland | 0.34 |
| S116 Adrenal gland | 0.34 |
| S117 Universal h- ref | 0.03 |
| S119 Breast | 0.00 |
| S120 Stomach | 0.05 |
| S121 Fetal Kidney | 0.54 |
| S122 Eye | 0.08 |
| S123 Mammary gland | 0.04 |
| S124 Ovary | 0.02 |
| S125 Pituitary gland | 0.22 |
| S127 human lupus liver | 0.05 |
| S128 human lupus Lung | 0.16 |
| S129 human lupus Spleen | 0.08 |
| S130 human lupus Kidney | 0.03 |
| S131 cirrhosis liver | 0.03 |
| S132 cirrhosis Lung | 0.00 |
| S133 cirrhosis Spleen | 0.02 |
| S134 cirrhosis Small intestine | 0.13 |
| S135 kidney Tumor | 0.14 |
| S136 Liver Tumor | 0.02 |
| S137 Lung Tumor | 2.56 |
| S142 Fetal Liver | 0.02 |
| S138 colon Tumor | 0.06 |
| S140 Fetal brain | 0.06 |
| S141 Fetal spleen | 0.03 |
| S139 Breast Tumor | 0.01 |
| S143 Fetal Heart | 0.01 |
| S11 mixed RA2 | 0.10 |
| S7 Fibroblast SSCA2 | 0.09 |
| S6 Fibroblast SSc N2 | 0.03 |
| S5 Fibroblast NF2 | 0.21 |
| S4 Fibroblast NF1 | 0.16 |
| S3 Fibroblast Clark N | 0.00 |
| S2 Fibroblast Howard ab | 0.04 |
| S1 Fibroblast AG1518 | 0.02 |
| S151 Disease Brain | 0.46 |
| S150 Throast | 0.02 |
| S149 Blood vessy Artery | 0.03 |
| S148 Appendix | 0.06 |
| S147 Bladder | 0.03 |
| S146 Adipose | 0.02 |
| S145 Lymph Node | 0.03 |
| S144 Fetal Lung | 0.00 |
| S67 mixed small intestine UC 18 | 0.06 |
| S65 mixed small intestine Crohn's 7 | 0.32 |
| S64 mixed small intestine Crohn's 8 | 0.41 |
| S63 mixed small intestine normal int 23 | 0.50 |
| S62 mixed small intestine normal int 21 | 0.37 |
| S52 mixed colon 13073 | 0.03 |
| S50 mixed colon 13224 | 0.21 |
| S29 mixed Lung D | 0.11 |
| S28 mixed Lung C | 0.08 |
| S27 mixed Lung A | 0.06 |
| S19 mixed OA4 | 1.52 |
| S18 Fibroblast LA13 | 0.02 |
| S17 Fibroblast LN14 | 0.02 |
| S16 Fibroblast LAb1 | 0.02 |
| S15 Fibroblast LN1 | 0.05 |
| S13 mixed OA1 | 1.55 |
| S12 mixed RA3 | 0.03 |
| BN5 atherosclerotic plaque Z3 | 0.05 |
| BN3 atherosclerotic plaque Z2 | 0.05 |
| BN1 atherosclerotic plaque Z1 | 0.00 |
| S20 Keratinocytes skin K1 | 0.05 |
| S21 Keratinocytes skin K2 | 0.64 |
| S25 LDC lung | 0.21 |
| S36 THP-1 mono/mac | 0.90 |

Defining a threshold of Expression level of INSP163 relative to GAPDH expression of 1.0, TaqMan expression results show unexpected restricted expression of INSP163 in one lung tumor tissue and in two osteoarthritis tissues. No expression of INSP163 is seen in normal lung tissues (expression level of INSP163 relative to GAPDH is below 0.12). The lung tumor is a bronchogenic carcinoma, more specifically a squamous cell carcinoma.

This specific pattern of expression leads to the conclusion of the involvement of INSP163 in lung cancer and osteoarthritis. These surprising properties characterizing the polynucleotides or the corresponding polypeptides of International Application No. PCT/GB2004/004544 make them particularly suitable for the preparation of a drug or pharmaceutical composition.

### Example 3 - Assay for determining IKK2 activity in Non-Small Cell Lung Carcinoma Cells (A549)

Tumor necrosis factor-α (TNFα.) is a pleiotropic cytokine with multifunctions including cell activation, differentiation and apoptosis. TNFα exerts both apoptotic and antipoptotic effects in cell-type specific manner. The antiapoptotic effects of TNFα appears to be mediated by the upregulation of NF-κB activity. TNFα induced activation of NF-κB increases the expression of several antiapoptotic proteins that protect cells from cell death. When this pathway is inhibited, TNFα, can potentially induce cell death. Activation of NF-kB is mediated by IKK complex.

An assay has been developed that can measure the activation of IKK2 activity in A549 cells, a human lung carcinoma cell. Since TNFα can induce both pro- and anti-apoptotic pathway, blocking the anti-apoptotic gene expression by cycloheximide can lead to cell death. Once the cells undergo apoptosis, they detach from the culture surface. Upon fixing the cells with crystal violet followed by washing, only live cells are stained and this could be read at 540 nm. Thus, this measure is used to determine cell death in A549 cells.

When A549 cells are treated with TNFα in the presence of cycloheximide, it results in apoptosis. Upon pretreating cells with IL-1β or TNFα, to induce the IKK pathway, thus upregulate the anti-apoptotic genes, it can protect cells from death induced by TNFα+cycloheximide treatment. During the pretreatment step with IL-1β, blocking IKK activity with a specific inhibitor can abolish the protective effect of IL1β on TNFα+cycloheximide mediated cell death. Thus, this property of TNFα signaling is used to monitor IKK activity in A549 cells. The IKK- inhibitor can dose-dependently block IL-1β-mediated protective effect in A549 cells, while with EGF there is no protective effect. The protocol followed for monitoring IKK activity is as follows:
1) A549 Cells are seeded (50,000 cells/well) and cultured overnight,
2) The cells are pretreated with IL-1β (1 ng/ml) or TNF-α with or without the compound, i.e. INSP163 polypeptide, in serum free media for overnight. The compound is a specific inhibitor of IKK activity,
3) The cells are treated with TNF-α and cycloheximide for 8 hours, and
4) Cell death is monitored with crystal violet.

The compound, i.e. INSP163, will induce cell death of non-small cell lung carcinoma cell (A549 cells) in the above-mentioned assay. It is therefore concluded that INSP163 (INSP163 polypeptide) is useful for the treatment and/or prevention of cancer, preferably lung cancer, preferably bronchogenic carcinoma.

### REFERENCES

1. Abraham DJ, Shiwen X, Black CM, Sa S, Xu Y, Leask A.J Biol Chem. 2000 May 19;275(20):15220-5.
2. Altschul S F et al, J Mol Biol, 215, 403-410, 1990
3. Altschul S F et al, Nucleic Acids Res., 25:389-3402, 1997
4. Devereux J et al, Nucleic Acids Res, 12, 387-395, 1984.
5. Engelmann, H., Novick, D., and Wallach, D., 1990, J.Biol.Chem. 265, 1531-1536.
6. Pearson W R, Methods in Enzymology, 183, 63-99, 1990
7. Pearson W R and Lipman D J, Proc Nat Acad Sci USA, 85, 2444-2448,1988.
8. Tucci, A., James, H., Chicheportiche, R., Bonnefoy, J.Y., Dayer, J.M., and Zubler, R.H., 1992, J.Immunol. 148, 2778-2784.

## Claims

1. A polypeptide for use in a method of treatment and/or prevention of lung cancer, wherein said polypeptide is selected from the group consisting of:
a) A polypeptide consisting of SEQ ID NO: 30, or
b) A polypeptide comprising any of SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 30 or SEQ ID NO: 34, or
c) A glycosylated form of the polypeptide of any of (a) or (b), wherein the polypeptide is glycosylated at one or more sites, or
d) A salt or a fusion protein of any of (a) to (c).

2. A nucleic acid molecule for use in a method of treatment and/or prevention of a lung cancer, wherein said nucleic acid is selected from the group consisting of:
a) A nucleic acid sequence as set forth in any of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 29 or SEQ ID NO: 33 or
b) A nucleic acid sequence of (a) wherein said nucleic acid sequence encodes an amino acid sequence having conservative amino acid substitutions to the amino acid sequences in SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 30 or SEQ ID NO: 34.

3. Pharmaceutical composition for use in a method of treatment and/or prevention of lung cancer comprising a polypeptide selected from the group consisting of:
a) A polypeptide consisting of SEQ ID NO: 30, or
b) A polypeptide comprising any of SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 30 or SEQ ID NO: 34, or
c) A glycosylated form of the polypeptide of any of (a) or (b), wherein the polypeptide is glycosylated at one or more sites, or
d) A salt or a fusion protein of any of (a) to (c).

4. Pharmaceutical composition for use in a method of treatment and/or prevention of a lung cancer comprising a nucleic acid selected from the group consisting of:
a) A nucleic acid sequence as set forth in any of SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 29 or SEQ ID NO: 33, or
b) A nucleic acid sequence of (a) wherein said nucleic acid sequence encodes an amino acid sequence having conservative amino acid substitutions to the amino acid sequences in SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 30 or SEQ ID NO: 34.

5. The polypeptide for the use according to claim 1 or the pharmaceutical composition for the use according to claim 3, wherein the fusion protein comprises an immunoglobulin (Ig) fusion.

6. The polypeptide or the pharmaceutical composition for the use according to claim 5, wherein the 1g fusion is an Fc fusion.

7. The nucleic acid for the use according to claim 2 or the pharmaceutical composition for the use according to claim 4, wherein the nucleic acid molecule is comprised in an expression vector.

8. The nucleic acid or the pharmaceutical composition for the use according to claim 7, wherein the vector is a gene therapy vector.

9. Use of a vector comprising a nucleic acid molecule according to claim 2, for inducing and/or enhancing the endogenous production of a polypeptide according to claim 1 in a cell for the preparation of a medicament or a pharmaceutical composition for the treatment and/or prevention of lung cancer.

10. Use of a cell comprising a nucleic acid molecule according to claim 2 for the preparation of a medicament or a pharmaceutical composition for the treatment and/or prevention of lung cancer.

11. Use of a cell expressing a polypeptide according to claim 1 for the preparation of a medicament or a pharmaceutical composition for the treatment and/or prevention of lung cancer.

12. The pharmaceutical composition for the use according to any one of claims 3 to 8, wherein the pharmaceutical composition further comprises an anti-cancer agent for simultaneous, sequential, or separate use.

13. The pharmaceutical composition for the use according to claim 12, wherein the anti-cancer agent is selected from the group consisting of platinum compounds such as cisplatin and carboplatin, vinca alkaloids such as vinorelbine, vincristine, and vinblastine, taxines such as docetaxel and paclitaxel, or various topoisomerase inhibitors.

14. The polypeptide, the nucleotide or the pharmaceutical composition for the use according to any of claims 1-8 and 12-13 or the use according to claim 9-11, wherein the lung cancer is bronchogenic carcinoma, bronchial carcinoid, chondromatous hamartoma, solitary lymphoma, sarcoma, multifocal lymphomas, squamous cell carcinoma, undifferentiated small cell carcinoma, undifferentiated large cell carcinoma, adenocarcinoma, Bronchioloalveolar carcinoma or non-small cell lung carcinoma

## Patentansprüche

1. Polypeptid zur Verwendung in einem Verfahren zur Behandlung und/oder Prävention von Lungenkrebs, wobei das Polypeptid aus der Gruppe, bestehend aus:
(a) einem Polypeptid, bestehend aus SEQ ID NO: 30 oder
(b) einem Polypeptid, umfassend irgendeine der SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 30 oder SEQ ID NO: 34, oder
(c) einer glykosylierten Form des Polypeptids aus irgendeinem von (a) oder (b), wobei das Polypeptid an einer oder mehreren Stellen glykosyliert ist, oder
(d) einem Salz oder einem Fusionsprotein von irgendeinem von (a) bis (c),
ausgewählt ist.

2. Nukleinsäuremolekül zur Verwendung in einem Verfahren zur Behandlung und/oder Prävention eines Lungenkrebs, wobei die Nukleinsäure aus der Gruppe, bestehend aus:
(a) einer Nukleinsäuresequenz, wie in irgendeiner der SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 29 oder SEQ ID NO: 33 dargelegt, oder
(b) einer Nukleinsäuresequenz aus (a), wobei die Nukleinsäuresequenz eine Aminosäuresequenz kodiert, die konservative Aminosäuresubstitutionen gegenüber den Aminosäuresequenzen in den SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 30 oder SEQ ID NO: 34 aufweisen, ausgewählt ist.

3. Pharmazeutische Zusammensetzung zur Verwendung in einem Verfahren zur Behandlung und/oder Prävention von Lungenkrebs, umfassend ein Polypeptid, das aus der Gruppe, bestehend aus:
(a) einem Polypeptid, bestehend aus SEQ ID NO: 30, oder
(b) einem Polypeptid, umfassend irgendeine der SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 30 oder SEQ ID NO: 34, oder
(c) einer glykosylierten Form des Polypeptids aus irgendeinem von (a) oder (b), wobei das Polypeptid an einer oder mehreren Stellen glykosyliert ist, oder
(d) einem Salz oder einem Fusionsprotein von irgendeinem von (a) bis (c),
ausgewählt ist.

4. Pharmazeutische Zusammensetzung zur Verwendung in einem Verfahren zur Behandlung und/oder Prävention eines Lungenkrebs, umfassend eine Nukleinsäure, die aus der Gruppe, bestehend aus:
(a) einer Nukleinsäuresequenz, wie in irgendeiner der SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 29 oder SEQ ID NO: 33 dargelegt, oder
(b) einer Nukleinsäuresequenz aus (a), wobei die Nukleinsäuresequenz eine Aminosäuresequenz kodiert, die konservative Aminosäuresubstitutionen gegenüber den Aminosäuresequenzen in den SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 30 oder SEQ ID NO: 34 aufweisen, ausgewählt ist.

5. Polypeptid zur Verwendung gemäß Anspruch 1 oder pharmazeutische Zusammensetzung zur Verwendung gemäß Anspruch 3, wobei das Fusionsprotein eine Immunoglobulin- (Ig) Fusion umfasst.

6. Polypeptid oder pharmazeutische Zusammensetzung zur Verwendung gemäß Anspruch 5, wobei die Ig-Fusion eine Fc-Fusion ist.

7. Nukleinsäure zur Verwendung gemäß Anspruch 2 oder pharmazeutische Zusammensetzung zur Verwendung gemäß Anspruch 4, wobei das Nukleinsäuremolekül in einem Expressionsvektor enthalten ist.

8. Nukleinsäure oder pharmazeutische Zusammensetzung zur Verwendung gemäß Anspruch 7, wobei der Vektor ein Gentherapie-Vektor ist.

9. Verwendung eines Vektors, der ein Nukleinsäuremolekül gemäß Anspruch 2 umfasst, um die endogene Herstellung eines Polypeptids gemäß Anspruch 1 in einer Zelle zu induzieren und/oder zu verstärken, zur Herstellung eines Medikaments oder einer pharmazeutischen Zusammensetzung zur Behandlung und/oder Prävention von Lungenkrebs.

10. Verwendung einer Zelle, die ein Nukleinsäuremolekül gemäß Anspruch 2 umfasst, zur Herstellung eines Medikaments oder einer pharmazeutischen Zusammensetzung zur Behandlung und/oder Prävention von Lungenkrebs.

11. Verwendung einer Zelle, die ein Polypeptid gemäß Anspruch 1 exprimiert, zur Herstellung eines Medikaments oder einer pharmazeutischen Zusammensetzung zur Behandlung und/oder Prävention von Lungenkrebs.

12. Pharmazeutische Zusammensetzung zur Verwendung gemäß einem der Ansprüche 3 bis 8, wobei die pharmazeutische Zusammensetzung weiterhin ein Antikrebsmittel umfasst, zur gleichzeitigen, aufeinanderfolgenden oder getrennten Verwendung.

13. Pharmazeutische Zusammensetzung zur Verwendung gemäß Anspruch 12, wobei das Antikrebsmittel aus der Gruppe, bestehend aus Platinverbindungen, wie z.B. Cisplatin und Carboplatin, Vincaalkaloiden, wie z.B. Vinorelbin, Vincristin und Vinblastin, Taxanen, wie z.B. Docetaxel und Paclitaxel, oder verschiedenen Topoisomeraseinhibitoren, ausgewählt ist.

14. Polypeptid, Nukleotid oder pharmazeutische Zusammensetzung zur Verwendung gemäß einem der Ansprüche 1-8 und 12-13 oder Verwendung gemäß Anspruch 9-11, wobei der Lungenkrebs bronchogenes Karzinom, Bronchialkarzinoid, chondromatöses Hamartom, solitäres Lymphom, Sarkom, multifokale Lymphome, Plattenepithelkarzinom, undifferentiertes kleinzelliges Karzinom, undifferentiertes großzelliges Karzinom, Adenokarzinom, Bronchioalveoläres Karzinom oder nicht-kleinzelliges Lungenkarzinom ist.

## Revendications

1. Polypeptide pour utilisation dans un procédé de traitement et/ou de prévention d'un cancer pulmonaire, lequel polypeptide est choisi dans l'ensemble formé par les suivants :
a) un polypeptide constitué de la Séquence N° 30,
b) ou un polypeptide comprenant n'importe laquelle des Séquences N° 2, N° 4, N° 6, N° 8, N° 10, N° 12, N° 14, N° 30 et N° 34,
c) ou une forme glycosylée de n'importe lequel des polypeptides (a) et (b), lequel polypeptide est glycosylé en un ou plusieurs site(s),
d) ou un sel ou une protéine de fusion de n'importe lequel des polypeptides (a) à (c).

2. Molécule d'acide nucléique pour utilisation dans un procédé de traitement et/ou de prévention d'un cancer pulmonaire, lequel acide nucléique est choisi dans l'ensemble formé par les suivants :
a) un acide nucléique présenté comme étant n'importe laquelle des Séquences N° 1, N° 3, N° 5, N° 7, N° 9, N° 11, N° 13, N° 29 et N° 33,
b) ou une séquence d'acide nucléique indiquée en (a), laquelle séquence d'acide nucléique code une séquence d'acides aminés qui présente des substitutions d'acides aminés conservatrices par rapport à l'une des séquences d'acides aminés des Séquences N° 2, N° 4, N° 6, N° 8, N° 10, N° 12, N° 14, N° 30 et N° 34.

3. Composition pharmaceutique conçue pour être utilisée dans un procédé de traitement et/ou de prévention d'un cancer pulmonaire, comprenant un polypeptide choisi dans l'ensemble formé par les suivants :
a) un polypeptide constitué de la Séquence N° 30,
b) ou un polypeptide comprenant n'importe laquelle des Séquences N° 2, N° 4, N° 6, N° 8, N° 10, N° 12, N° 14, N° 30 et N° 34,
c) ou une forme glycosylée de n'importe lequel des polypeptides (a) et (b), lequel polypeptide est glycosylé en un ou plusieurs site(s),
d) ou un sel ou une protéine de fusion de n'importe lequel des polypeptides (a) à (c).

4. Composition pharmaceutique conçue pour être utilisée dans un procédé de traitement et/ou de prévention d'un cancer pulmonaire, comprenant un acide nucléique choisi dans l'ensemble formé par les suivants :
a) un acide nucléique présenté comme étant n'importe laquelle des Séquences N° 1, N° 3, N° 5, N° 7, N° 9, N° 11, N° 13, N° 29 et N° 33,
b) ou une séquence d'acide nucléique indiquée en (a), laquelle séquence d'acide nucléique code une séquence d'acides aminés qui présente des substitutions d'acides aminés conservatrices par rapport à l'une des séquences d'acides aminés des Séquences N° 2, N° 4, N° 6, N° 8, N° 10, N° 12, N° 14, N° 30 et N° 34.

5. Polypeptide pour utilisation conforme à la revendication 1, ou composition pharmaceutique pour utilisation conforme à la revendication 3, la protéine de fusion comportant une immunoglobuline (Ig) fusionnée.

6. Polypeptide ou composition pharmaceutique pour utilisation, conforme à la revendication 5, l'immunoglobuline fusionnée étant un fragment Fc fusionné.

7. Acide nucléique pour utilisation conforme à la revendication 2, ou composition pharmaceutique pour utilisation conforme à la revendication 4, la molécule d'acide nucléique étant incluse dans un vecteur d'expression.

8. Acide nucléique ou composition pharmaceutique pour utilisation, conforme à la revendication 7, le vecteur étant un vecteur pour thérapie génique.

9. Utilisation d'un vecteur comprenant une molécule d'acide nucléique conforme à la revendication 2 pour induire et/ou augmenter la production endogène, dans une cellule, d'un polypeptide conforme à la revendication 1, en vue de la préparation d'un médicament ou d'une composition pharmaceutique conçu(e) pour le traitement et/ou la prévention d'un cancer pulmonaire.

10. Utilisation d'une cellule comprenant une molécule d'acide nucléique conforme à la revendication 2, en vue de la préparation d'un médicament ou d'une composition pharmaceutique conçu(e) pour le traitement et/ou la prévention d'un cancer pulmonaire.

11. Utilisation d'une cellule exprimant un polypeptide conforme à la revendication 1, en vue de la préparation d'un médicament ou d'une composition pharmaceutique conçu(e) pour le traitement et/ou la prévention d'un cancer pulmonaire.

12. Composition pharmaceutique pour utilisation conforme à l'une des revendications 3 à 8, laquelle composition pharmaceutique comprend en outre un agent anti-cancéreux, à utiliser simultanément, successivement ou séparément.

13. Composition pharmaceutique pour utilisation conforme à la revendication 12, pour laquelle l'agent anti-cancéreux est choisi dans l'ensemble formé par les composés du platine comme le cisplatine ou le carboplatine, les alcaloïdes vinca comme la vinorelbine, la vincristine ou la vinblastine, les taxines comme le docétaxel ou le paclitaxel, et divers inhibiteurs de topoisomérase.

14. Polypeptide, acide nucléique ou composition pharmaceutique pour utilisation, conforme à l'une des revendications 1 à 8, 12 et 13, ou utilisation conforme à l'une des revendications 9 à 11, le cancer pulmonaire étant un cancer broncho-pulmonaire, un carcinoïde des bronches, un hamartome chondromateux, un lymphome solitaire, un sarcome, un lymphome à foyers multiples, un carcinome épidermoïde, un carcinome à petites cellules indifférencié, un carcinome à grandes cellules indifférencié, un adénocarcinome, un cancer bronchio-alvéolaire, ou un cancer pulmonaire non à petites cellules.
